# EUROPÄISCHE PATENTSCHRIFT

(11) **EP 3 151 878 B1**
(45) Veröffentlichungstag und Bekanntmachung des Hinweises auf die Patenterteilung: **06.05.2020**
(21) Anmeldenummer: 15726175.1
(22) Anmeldetag: 03.06.2015
(51) Int. Cl.: A61M 1/16, A61M 16/00, A61M 16/10

(54) **BEATMUNGSSYSTEM MIT MASCHINELLER BEATMUNG UND EXTRAKORPORALEM BLUTGASAUSTAUSCH**
VENTILATION SYSTEM WITH MECHANICAL VENTILATION AND EXTRACORPOREAL BLOOD GAS EXCHANGE
SYSTÈME D'ASSISTANCE RESPIRATOIRE COMPRENANT UNE ASSISTANCE RESPIRATOIRE ARTIFICIELLE ET UNE ASSISTANCE CIRCULATOIRE EXTRACORPORELLE POUR L'OXYGÉNATION DU SANG

(30) Priorität: 05.06.2014 DE 102014107980
(43) Veröffentlichungstag der Anmeldung: 12.04.2017
(73) Patentinhaber: Hamilton Medical AG, 7402 Bonaduz (CH)
(72) Erfinder: LAUBSCHER, Thomas, 7403 Rhäzüns (CH); NOVOTNI, Dominik, 7000 Chur (CH)
(74) Vertreter: Schmitt-Nilson Schraud Waibel Wohlfrom Patentanwälte Partnerschaft mbB
(86) Internationale Anmeldenummer: PCT/EP2015/062369
(87) Internationale Veröffentlichungsnummer: WO 2015/185618

(56) Entgegenhaltungen:
- WO-A1-2011/021978
- US-A- 5 810 759
- US-A1- 2008 041 381

## Beschreibung

Die vorliegende Erfindung betrifft ein Beatmungssystem, umfassend eine Einrichtung zur maschinellen Beatmung, insbesondere Überdruckbeatmung, der Lunge eines Patienten und zum anderen eine ECLS-Einrichtung zum extrakorporalen Blutgasaustausch.

US 5 810 759 A zeigt ein Steuersystem zum Regeln der Sauerstoff- und Kohlendioxidkonzentration von Blut in einem an einen Patienten angeschlossenen ECLS-Kreislauf. Das Steuersystem enthält einen Gasanalysator, der die Konzentrationen von Kohlendioxid im Blut bestimmt, das dem Patienten entnommen wird, und von Sauerstoff im Blut, das dem Patienten zurückgeführt wird. Der Gasanalysator liefert Signale entsprechend der Konzentrationen an eine Steuerung. Der Steuerung werden Sollwerte für diese Konzentrationen vorgegeben. Unter Verwendung eines Regelalgorithmus berechnet die Steuerung die Gasflussregelungs- und Blutflussregelungssignale basierend auf den Differenzen der Konzentrationssignale und der Sollwerte. Eine mit der Steuerung gekoppelte Gasströmungssteuerung empfängt ein Gasströmungssteuersignal, Gas von einer Gasquelle mit einer vorbestimmten Rate an den ECLS-Kreislauf zu liefern.

Das System ist dazu ausgebildet, eine maschinelle Atmungsunterstützung durch die Einrichtung zur maschinellen Beatmung einerseits und extrakorporalen Blutgasaustausch, insbesondere Oxygenierung/Ventilation, durch die ECLS-Einrichtung andererseits in koordinierter Weise automatisiert durchzuführen, um den Gasaustausch im Blutkreislauf des Patienten zu unterstützen. Das System ist speziell für die Intensivbehandlung von Patienten ausgebildet, und zwar insbesondere zur Unterstützung der Lungenfunktion bzw. des Blutgasaustauschs, wenn im Verlauf einer Intensivbehandlung mit maschineller Beatmung eines Patienten eine Entwicklung hin zu Zuständen eintritt, in welchen mittels maschineller Beatmung alleine keine ausreichende Unterstützung der Lungenfunktion des Patienten mehr erreichbar ist oder in welchen eine ausreichende Unterstützung der Lungenfunktion die Einstellung von Beatmungsparametern für die maschinelle Beatmung erforderlich machen würde, bei denen Schädigungen von Lunge und Atemapparat und/oder des Herz-Kreislauf-Systems drohen.

Die ECLS-Einrichtung gibt erfindungsgemäß ein Niveau des extrakorporalen Blutgasaustauschs, insbesondere der Oxygenierung/Ventilation, vor und die Einrichtung zur maschinellen Beatmung stellt sich anhand des von der ECLS-Einrichtung vorgegebenen Niveaus des extrakorporalen Gasaustauschs automatisiert auf ein Niveau der maschinellen Atmungsunterstützung ein.

Die vorgeschlagene Einrichtung zur maschinellen Beatmung kann , wie das heutzutage für maschinelle Beatmung üblich ist, als Überdruckbeatmungseinrichtung realisiert sein. Bei Überdruckbeatmung wird der Atemweg beim Einatmen durch einen externen Überdruck beaufschlagt, der dafür sorgt, dass ggf. mit Sauerstoff und/oder anderen Beimengungen versehene Luft in die Lungen gepresst wird. Das Ausatmen erfolgt in den meisten Fällen passiv durch Beaufschlagen des Atemwegsausgangs mit Umgebungsdruck, wobei sich der Druck in der Lunge gegenüber dem Umgebungsdruck entspannt. Ggf. kann auch beim Ausatmen eine gewisse maschinelle Unterstützung erfolgen. Der eigentliche Austausch von Blutgasen, insbesondere die Anreicherung von venösem Blut mit O2 bzw. die Abreicherung von CO2 aus dem venösen Blut, findet bei der maschinellen Beatmung grundsätzlich innerhalb der Lunge statt. Die Einrichtung zur maschinellen Beatmung ermöglicht eine Vielzahl unterschiedlicher Beatmungsmodi, die von Formen der assistierten Spontanatmung bis zu Formen der vollständig maschinenkontrollierten Beatmung reichen. Dabei kann die Einrichtung zur maschinellen Beatmung eine kontinuierliche Adaption der Invasivität der Beatmung, beispielsweise durch Wechsel von Beatmungsmodi, entsprechend dem Zustand des Patienten erlauben.

Der Zustand des Patienten kann durch unterschiedliche Sensoren bzw. Messprozeduren erfasst werden, die jeweils bestimmte Parameter erfassen, beispielsweise die in der maschinellen Beatmung übliche Bestimmung des Anteils von O2 bzw. CO2 in der eingeatmeten Luft bzw. in der ausgeatmeten Luft (z.B. als PetCO2 am Ende der Expirationsphase), oder Messung der Sauerstoffsättigung im Blut mittels Pulsoxymetrie (SpO2), aber auch weitergehend die Messung des Widerstands (Resistance) und der Dehnbarkeit (Compliance) der Lunge. Es können sogar Größen erfasst werden, die in der Beatmung üblicherweise nur sporadisch bzw. in manueller Weise erfasst werden, insbesondere der Anteil von Atemgasen im Blut durch entsprechende chemische Analyse (PaO2, PaCO2) oder auf optischem Wege. All diese Messprozeduren können in automatisierter Weise erfolgen, d.h. ohne dass Eingriffe durch Ärzte oder Pflegepersonal erforderlich wären.

Die Einrichtung zur maschinellen Beatmung arbeitet nach bestimmten Beatmungsparametern. Hierzu zählen u.a. die Sauerstoffkonzentration in der der Lunge zugeführten Atemluft (FiO2); die Atemfrequenz, d.h. die Anzahl der Atemzüge pro Minute; das Tidalvolumen (auch Atemzugvolumen genannt), d.h. das Volumen an Luft, das pro Atemzug in die Lunge appliziert werden soll; der Inspirationsfluss, d.h. der Fluss der Luft während der Inspirationsphase (dieser kann - und wird in der Regel - durchaus auch während einer einzigen Inspirationsphase variieren); der maximale Inspirationsdruck, d.h. der maximale Druck der Luft am Atemwegseingang während der Inspirationsphase; der positive endexpiratorische Druck (sog. PEEP), d.h. ein Überdruck, mit dem der Atemwegseingang während der Beatmung dauerhaft beaufschlagt wird, um einem Kollabieren von Teilen der Lungenbläschen am Ende der Expiration entgegenzuwirken. Der PEEP lässt sich im Allgemeinen als Druck am Atemwegsausgang am Ende der Expirationsphase bestimmen.

Die Einrichtung zur maschinellen Beatmung kann eine Beatmung auf Grundlage von fest voreingestellten Beatmungsparametern, etwa FiO2, PEEP oder maximaler Inspirationsdruck, durchführen. Andere Beatmungsparameter können durch die Beatmungseinrichtung jeweils automatisch angepasst werden, um einen bestmöglichen Beatmungszustand zu erzielen. Dabei kann die Beatmung derart erfolgen, dass die Einrichtung zur maschinellen Beatmung für einen gegebenen Beatmungsmodus anhand fest vorgegebener Beatmungsparameter sowie automatisiert erfasster flexibler Beatmungsparameter die relevanten flexiblen Beatmungsparameter automatisch einstellt (beispielsweise mittels jeweiliger Regelkreise). Die Art und Anzahl von fest vorgegebenen Beatmungsparametern und einzustellenden flexiblen Beatmungsparametern ist unterschiedlich für unterschiedliche Beatmungsmodi wie druckkontrollierte Beatmung, volumenkontrollierte Beatmung, BiPAP-Beatmung, um nur einige zu nennen. Je weniger Beatmungsparameter fest vorgegeben werden, desto flexibler kann die Beatmungseinrichtung auf unterschiedliche Einflüsse reagieren und desto weniger manuelle Eingriffe in die Beatmung sind erforderlich. Allerdings steigt der zur Regelung erforderliche Aufwand auch entsprechend an und es besteht insbesondere die Gefahr, dass sich Kombination von bestimmten Beatmungsparametern einstellen, die zu Schädigungen der Lunge führen. Um das zu vermeiden, können für die flexiblen Beatmungsparameter bestimmte Rahmenbedingungen vorgegeben werden. Die jeweiligen Beatmungsparameter können dann nur innerhalb der für sie festgelegten Rahmenbedingungen durch die Beatmungseinrichtung variiert werden.

Es ist auch denkbar, dass die Einrichtung zur maschinellen Beatmung zwischen verschiedenen Beatmungsmodi wechseln kann bzw. einen jeweils geeigneten Beatmungsmodus aus einer Mehrzahl von Beatmungsmodi auswählen kann. Dies kann in automatisierter Weise geschehen durch Auswertung von jeweils erfassten Beatmungsparametern bzw. den Beatmungszustand kennzeichnenden Größen. Als Beispiel sei verwiesen auf die unter der Bezeichnung ASV (Adaptive Support Ventilation) bekannten Beatmungssysteme der Anmelderin, über die beispielsweise die unter dem Namen _{"}S1" vertriebene Einrichtung zur maschinellen Beatmung der Anmelderin verfügt.

Neben maschineller Beatmung existieren bestimmte Verfahren zur extrakorporalen Lungenunterstützung (ECLS=extracorporal lung support), bei denen die Funktion der Lungen teilweise oder vollständig durch eine Maschine übernommen wird, in dem Sinne, dass der Austausch von Blutgasen, d.h. die Anreicherung des Blutes mit Sauerstoff und/oder die Entfernung von CO2 aus dem Blut, maschinell unterstützt wird oder sogar vollständig maschinell erfolgt. ECLS ist eine in aller Regel intensivmedizinische Technik, ähnlich der bei Operationen am Herzen häufig eingesetzten Herz-Lungen-Maschine. Während bei der Herz-Lungen-Maschine auch die kardialen Funktionen zur Erhaltung des Blutkreislaufs vollständig von der Maschine übernommen werden müssen, stehen bei ECLS in aller Regel - wenn auch nicht ausschließlich - der Ersatz bzw. die Unterstützung der Lungenfunktion im Vordergrund. Venös-Venös arbeitende ECLS-Systeme unterstützen dabei in aller Regel die Lungenfunktion, indem Blut aus dem venösen System entnommen und nach extrakorporal erfolgtem Blutgasaustausch wieder in das venöse System zurückgeführt wird. Venös-arteriell arbeitende ECLS-Systeme, bei denen das aus dem venösen System entnommene Blut nach extrakorporal erfolgtem Blutgasaustausch in das arterielle System zurückgeführt wird, bieten darüber hinaus auch die Möglichkeit einer maschinellen Unterstützung der kardialen Funktionen. Im Zusammenhang mit der vorliegenden Erfindung können sowohl venös-venös arbeitende ECLS-Systeme als auch venör-arteriell arbeitende ECLS-Systeme eingesetzt werden. ECLS wird angewendet bei Patienten, deren Lungen so schwer geschädigt sind, dass die Lungenbläschen selbst den Gasaustausch nicht mehr in dem Maß ermöglichen, um die Atemfunktion sicherzustellen. ECLS ist damit eine Form der extrakorporalen Organersatzverfahren.

Bei den meisten ECLS Verfahren, beispielsweise bei der sog.extrakorporalen Membranoxygenierung (ECMO), werden Kanülen in zwei große Blutgefäße (entweder eine oder zwei große Venen bei venös-venösen Varianten oder eine große Vene und eine Arterie bei venös-arteriellen Varianten) eingebracht, um Blut kontinuierlich durch einen extrakorporalen Oxygenator, z.B. einen Membran-Oxygenator im Falle der ECMO (ECMO = extrakorporale Membranoxygenierung), zu pumpen, der CO2 aus dem Blut entfernt und das Blut mit O2 anreichert, und das so aufbereitete Blut dann in den Blutkreislauf des Patienten zurückzuführen. Dabei kann das Blut in das venöse System des Patienten zurückgeführt werden (sog. veno-venöse ECLS), so dass die kardiale Funktion weiterhin vom Herzen übernommen wird. Es gibt aber auch Formen, bei denen auch das Herz überbrückt wird und das Blut ins arterielle System stromabwärts des Herzens zurückgeführt wird, um die kardiale Funktion mittels des Pumpkreislaufs zu unterstützen (sog. veno-arterielle ECLS). Alternativ zu einem MembranOxygenator können auch andere Formen verwendet werden, beispielsweise Bläschen-Oxygenatoren. In manchen Fällen kann ECLS minimalinvasiv durchgeführt werden, beispielsweise durch Einsatz eines Gasaustauschkatheters (IGEC=integrated gas exchange catheter) direkt in eine große Vene. Dann braucht dem Patienten kein Blut entnommen zu werden, da der Gasaustausch in der Vene durch die O2 zuführenden bzw. CO2 abführenden Kapillaren des Katheters erfolgt.

ECLS kann über Tage oder Wochen eine ausreichende Oxygenierung bzw. Ventilation gewährleisten und gibt damit der Lunge Zeit, ohne aggressive Beatmung zu heilen. Trotzdem wird ECLS wegen der hohen technischen und personellen Anforderungen, Kosten und Komplikationsrisiken (z. B. Blutungen) als eine letzte Therapiemöglichkeit betrachtet.

In aller Regel können bei ECLS folgende Parameter beeinflusst werden: Zusammensetzung des dem extrakorporalen Oxygenator zugeführten Gases (beispielsweise der Anteil an O2 bzw. CO2), Fluss des dem extrakorporalen Oxygenator-zugeführten Gases, Fluss von Blut durch den exkorporalen Oxygenator.

Die Erfindung befasst sich mit einem Beatmungssystem, bei dem maschinelle Beatmung und ECLS koordiniert gemeinsam betrieben werden sollen, um eine möglichst effiziente maschinelle Beatmung bei nur geringen schädigenden Auswirkungen auf die Lunge zu erzielen. Das Beatmungssystem soll eine Einrichtung zur maschinellen Beatmung und eine ECLS-Einrichtung aufweisen, die jeweils für sich grundsätzlich in einer Weise ausgebildet sein können wie oben beschrieben.

Aus der US 5 810 759 ist ein System bekannt, das eine automatische Regelung der ECMO-Parameter während veno-venöser ECMO vorsieht mit dem Ziel, den Austausch von CO2 durch O2 zu verbessern. Dabei können jeweils vorgegebene O2- und CO2-Konzentrationen im Blut durch Regelung des im ECMO-Kreis zirkulierenden Blutstroms und dazu proportional des dem durch den ECMO-Kreis zirkulierenden Blut zugeführten Oxygenator-Gasstroms eingeregelt werden. Ganz allgemein wird darauf hingewiesen, dass zusätzliche externe Parameter, z.B. Einstellungen einer maschinellen Beatmungseinrichtung, erfasst werden können und die Einstellung der Parameter des ECMO-Systems solche zusätzlichen Parameter berücksichtigen kann. Es wird über ein Tierexperiment berichtet, bei dem sich gezeigt hat, dass der ECMO-Regelkreis in der Lage ist, sich auf drastisch veränderte äußere Parameter - in dem Experiment die drastische Verringerung einer vorgegebenen Atmungsunterstützung durch maschinelle Überdruckbeatmung auf ein sehr niedriges Niveau - einzustellen.

Aus der WO 2011/021978 A1 ist ein Beatmungssystem bekannt, bei dem ECLS und Überdruckbeatmung kombiniert und in möglichst automatisierter Weise gemeinsam zur Unterstützung des Blutgasaustauschs bei einem Patienten betrieben werden sollen. Ziel soll sein, ECLS und Überdruckbeatmung auf einem jeweils optimalen Niveau einzusetzen, um Schädigungen am pulmonalen System durch zu intensive Überdruckbeatmung zu vermeiden. ECLS und Überdruckbeatmung besitzen jeweils eine eigene Steuerung zur Steuerung des extrakorporalen Blutgasaustauschs bzw. der Beatmung. Den Steuerungen ist je eine eigene Sensorik zugeordnet, die jeweilige Steuerparameter für ECLS bzw. die Überdruckbeatmung liefert. Die Steuerungen für ECLS und Überdruckbeatmung tauschen Daten aus, so dass wenigstens eine der Steuerungen ein Steuersignal für den ihr zugeordneten Prozess auf Grundlage wenigstens eines der anderen Steuerung zugeordneten Steuerparameters ausgibt. Einerseits soll dabei eine automatische Einstellung der Unterstützung durch ECLS erfolgen auf Grundlage von Steuerparametern, die von der Überdruckbeatmung geliefert werden: Für ein jeweiliges Niveau der Überdruckbeatmung wird anhand des CO2-Gehalts im Atemgas am Ende der Expirationsphase ein erforderliches Unterstützungsniveau durch ECLS eingestellt. Zum anderen soll eine automatische Einstellung der Unterstützung durch die Überdruckbeatmung erfolgen auf Grundlage von Steuerparametern, die vom ECLS geliefert werden: Für ECLS auf jeweiligem Niveau wird anhand des O2 Anteils im Blutkreislauf des Patienten das erforderliche Unterstützungsniveau durch die Überdruckbeatmung eingestellt. Allerdings zeigt sich, dass im Zusammenspiel der beiden Teilsysteme das Beatmungssystem anfällig ist für Instabilitäten und Komplikationen im Betrieb, weil sowohl das Teilsystem ECLS als auch das Teilsystem Überdruckbeatmung versuchen wird, einem zu niedrigen Grad an Unterstützung - ausgedrückt durch den jeweils vom anderen Teilsystem übergebenen Steuerparameter - entgegenzuwirken. Das führt zu Konkurrenz der beiden Teilsysteme mit der Tendenz, auf Veränderungen zu stark zu reagieren. Daher ist dieses System für einen weitgehend automatisierten koordinierten Betrieb beider Systeme im Rahmen einer Therapie kaum geeignet.

Erfindungsgemäß soll ein Beatmungssystem bereitgestellt werden, das in zuverlässigerer Weise eine vollautomatisch arbeitende Beatmung sicherstellt, und zwar insbesondere in Situationen, in denen maschinelle Beatmung und ECLS gleichzeitig angewendet werden, weil mittels maschineller Beatmung allein die Gefahr eines nicht nicht ausreichenden Blutgasaustauschs und/oder die Gefahr von irreparablen Schädigungen an Lunge, Atemapparat, oder Herz-Kreislaufsystem besteht. Dieses Beatmungssystem soll insbesondere ausgebildet sein zum Einsatz in der intensivmedizinischen Behandlung von Patienten mit fehlender oder zumindest unzureichender Atemfunktion, die eine maschinelle Beatmung erfordert.

Diese Aufgabe wird erfindungsgemäß gelöst durch ein Beatmungsystem gemäß beiliegender Ansprüche.

Bei der erfindungsgemäßen Kombination einer herkömmlich arbeitenden Einrichtung zur maschinellen Beatmung, insbesondere Überdruckbeatmung, mit einer Einrichtung zur extrakorporalen Lungenunterstützung ECLS (insbesondere einer Einrichtung zur extrakorporalen Membranoxygenierung ECMO), gibt die ECLS-Einrichtung durch Auswertung eines Parameters - das kann ein in der ECLS-Einrichtung bestimmter Parameter sein, z.B. der Anteil von O2 in dem durch den extrakorporalen Kreislauf fließenden Blut oder im venösen Blut stromabwärts der extrakorporalen Oxygenierung, aber auch ein von der maschinellen Beatmung gelieferter Parameter - vor, ob die Intensität der Behandlung durch ECLS gegenüber der Intensität der maschinellen Beatmung verändert werden soll. Der maschinellen Beatmung wird wenigstens eine den relativen Anteil der Intensität der ECLS-Behandlung anzeigende Größe als vorgegebener Parameter übergeben. Die maschinelle Beatmung stellt sich auf dann auf einen bestimmten Zustand ein, oder versucht jedenfalls sich so einzustellen, dass ein bestimmter Zustand erreicht wird. Damit dominiert grundsätzlich ECLS hinsichtlich des Therapieverlaufs über die maschinelle Beatmung. ECLS wird mit einem vorbestimmten Unterstützungsniveau vorgeben. Die maschinelle Beatmung stellt sich ein, wobei sie das Unterstützungsniveau des ECLS als vorgegeben nimmt.

Der weitere Therapieverlauf wird dadurch bestimmt, dass die ECLS-Einrichtung überprüft, inwieweit das eingestellte Unterstützungsniveau beibehalten bleiben soll oder verändert werden soll (in der Regel vermindert werden soll). Soll das Niveau der ECLS-Unterstützung vermindert werden, so wird ein das neue Niveau der ECLS-Unterstützung kennzeichnender Parameter an die Einrichtung zur maschinellen Beatmung übergeben als neue Vorgabe für die automatische Einstellung der maschinellen Beatmung. Dies kann beispielsweise in der Weise geschehen, dass die ECLS-Einrichtung einen Zielwert für das Niveau des extrakorporalen Blutgasaustauschs vorgibt. Die ECLS-Einrichtung verfügt über Regelmechanismen, die darauf hinarbeiten, dass die ECLS-Einrichtung unter Berücksichtigung aktuell erfasster Blutgaswerte sich dem vorgegebenen Zielwert anzunähern versucht und einen einmal erreichten Zielwert dann beibehält. Die maschinelle Beatmung stellt sich jeweils auf eine im Zuge dieser Annäherung an den Zielwert jeweils herrschenden Zustand der ECLS-Einrichtung ein und folgt somit mittelbar ebenfalls einer Entwicklung hin zu einem dem vorgegebenen Zielwert angepassten Beatmungszustand.

Eine mögliche Regelstrategie kann darin bestehen, dass ECLS mit anfänglich möglichst hohem Unterstützungsniveau eingestellt wird, insbesondere mit einem Unterstützungsniveau, bei dem die erforderliche Unterstützung der Lungenfunktion vollständig durch ECLS und ohne Unterstützung durch die maschinelle Beatmung geleistet werden kann (also mit ECLS Unterstützungsgrad von 100 %). Danach wird regelmäßig überprüft (in der Regel durch die ECLS-Steuerung), inwieweit der ECLS-Unterstützungsgrad verringert werden kann und der Unterstützungsgrad durch die maschinelle Beatmung entsprechend ansteigen kann.

Die Einrichtung zur maschinellen Beatmung versucht, für eine jeweils gegebene Einstellung der ECLS-Einrichtung, gekennzeichnet durch Parameter wie Fluss von Blut im ECLS-Kreislauf, gesamter Gasfluss von O2 bzw. anderen Gasen zum Oxygenator, Zusammensetzung des Oxygenator-Gases, die Parameter der maschinellen Beatmung so einzustellen, dass gegebene Zielwerte für den Gasaustausch erfüllt werden. In diesem Sinne bedeutet der erfindungsgemäße Vorschlag, dass ECLS "dominant" ist gegenüber der maschinellen Beatmung. Die Einrichtung zur maschinellen Beatmung sieht einen durch ECLS vorgegebenen Zustand als gegeben an und versucht, einen geeigneten Zustand der maschinellen Beatmung für diesen vorgegebenen Zustand einzustellen. Dabei ist die maschinelle Beatmung an bestimmte Vorgaben für die - für die abhängig vom jeweiligen Beatmungsmodus fest vorgegebenen Beatmungsparameter, jedoch durchaus auch an bestimmte Rahmenbedingungen für einstellbare Beatmungsparameter, wie maximaler PEEP, maximaler Atemwegsdruck oder maximales Minutenvolumen des zugeführten Beatmungsgases - gebunden. Das Minutenvolumen ergibt sich aus dem Produkt der Atemfrequenz mit dem in einem Atemzug jeweils applizierten Tidalvolumen.

Das Niveau des extrakorporalen Blutgasaustauschs durch die ECLS-Einrichtung kann automatisiert oder manuell vorwählbar sein. Vor allem interessant ist die Möglichkeit der automatisierten Vorgabe des Niveaus des extrakorporalen Blutgasaustauschs durch die ECLS-Einrichtung im Rahmen einer ECLS-Einstellstrategie, wie sie als Beispiel nachfolgend noch etwas genauer erläutert werden wird.

Die Einrichtung zur maschinellen Beatmung kann beispielsweise derart ausgelegt sein, dass sie für eine jeweils erfolgte Einstellung des Niveaus des extrakorporalen Blutgasaustauschs durch die ECLS-Einrichtung die Atmungsunterstützung durch maschinelle Beatmung automatisch steuert/regelt. Beispielsweise kann die Einrichtung zur maschinellen Beatmung dazu ausgebildet sein, in automatisierter Weise und im Rahmen vorgegebener Beatmungsparameter einen durch die Einrichtung zur maschinellen Beatmung einzustellenden Beatmungszustand auszuwählen und die Einrichtung zur maschinellen Beatmung derart zu steuern/regeln, dass sie den ausgewählten Beatmungszustand einnimmt oder jedenfalls einzunehmen versucht. Dies kann insbesondere in Form von maschineller Beatmung mit geschlossenem Regelkreis vorgesehen sein. Beatmungseinrichtungen, die eine ausreichende Flexibilität hinsichtlich des jeweils zu wählenden Beatmungsmodus bieten und die außerdem in der Lage sind, automatisch einen bestimmten geeignet erscheinenden Beatmungsmodus auszuwählen, sind beispielsweise solche, die über den unter dem Begriff _{"}ASV" (= Adaptive Support Ventilation) bekannten Beatmungsmodus verfügen. ASV bietet eine Beatmung mit geschlossenem Regelkreis und dynamischer Berechnung optimaler Beatmungsmodi sowie nach Auswahl eines Beatmungsmodus der noch erforderlichen automatischen Einstellungen der freien Parameter des jeweils ausgewählten Beatmungsmodus, so dass ausreichende Atmungsunterstützung bei möglichst geringen Auswirkungen auf den Patienten erreicht werden kann. Hierbei werden lungenschonende Behandlungsstragien, die bei möglichst geringen Drücken arbeiten, bevorzugt.

Die für die Einrichtung zur maschinellen Beatmung vorgegebenen Beatmungsparameter können sich aus dem durch die ECLS-Einrichtung vorgegebenen Niveau des extrakorporalen Blutgasaustauschs ableiten. Beispielsweise kann der maximale endexpiratorische Druck PEEPmax bei der maschinellen Beatmung oder der maximale Atemwegsdruck (PEEP + Pinsp)max bei der maschinellen Beatmung oder das maximale Minutenvolumen bei der maschinellen Beatmung vom jeweils vorgegebenen Niveau des extrakorporalen Blutgasaustauschs abhängen.

Man kann beispielsweise dem durch die ECLS-Einrichtung vorgegebenen Niveau des extrakorporalen Blutgasaustauschs einen Grad der extrakorporalen Unterstützung bei der Oxygenierung, d.h. bei der Anreicherung des Blutes mit Sauerstoff, zuordnen. Der Grad der extrakorporalen Unterstützung bei der Oxygenierung kann insbesondere ein relativer Wert sein, der sich auf den Anteil bezieht, den die extrakorporale Oxygenierung an der Anreicherung des Blutes mit Sauerstoff insgesamt hat, d.h. an der durch extrakorporale Oxygenierung und maschinelle Beatmung insgesamt bewirkten Anreicherung des Blutes mit Sauerstoff. Dieser Anteil soll im Folgenden auch als % ECLS_O2 bezeichnet werden. Der jeweilige Grad der extrakorporalen Unterstützung bei der Oxygenierung % ECLS_O2 kann dann einen maximalen positiven endexpiratorischen Druck PEEPmax für die maschinelle Beatmung festlegen. Der maximale positive endexpiratorische Druck (PEEPmax) kann mit abnehmendem Grad der extrakorporalen Unterstützung bei der Oxygenierung % ECLS_O2 ansteigen. Auf diese Weise wird dafür gesorgt, dass der maximale positive endexpiratorische Druck - der bei vielen Beatmungsmodi der maschinellen Beatmung eine Rahmenbedingung für die Intensität der Beatmung darstellt - immer weiter ansteigt, je geringer der Grad der extrakorporalen Unterstützung bei der Oxygenierung % ECLS_O2 wird. Damit steigt dementsprechend auch das Niveau der Unterstützung der Anreicherung von Blut mit O2 durch die maschinelle Beatmung im Vergleich zum Niveau der Unterstützung durch extrakorporale Oxygenierung. Die maschinelle Beatmung gewinnt mehr und mehr an Bedeutung je geringer der Grad der extrakorporalen Unterstützung bei der Oxygenierung wird. Die Funktion der Lunge als (natürliches) Organ zur Anreicherung des Blutes mit O2 gewinnt demgemäß immer mehr an Bedeutung, je länger die Kombination aus maschineller Beatmung und ECLS zufriedenstellend läuft. Dem entspricht, dass bei der bei der maschinellen Beatmung eingestellte positive endexpiratorische Druck PEEP immer größere Maximalwerte annehmen kann je länger die Kombination aus maschineller Beatmung und ECLS zufriedenstellend läuft. Höherer positiver endexpiratorischer Druck führt einerseits zu effizienterer maschineller Beatmung, weil ein Kollabieren von Lungenbläschen während der Expiration besser unterdrückt werden kann, bedeutet aber andererseits auch eine höhere Belastung des Lungengewebes.

Eine Veränderung des Niveaus des extrakorporalen Blutgasaustauschs ergibt sich mittelbar durch die erzwungene Veränderung des Anteils der maschinellen Beatmung gegenüber dem Anteil des extrakorporalen Blutgasaustauschs.

Eine Veränderung des Niveaus des extrakorporalen Blutgasaustauschs kann man auch unmittelbar dadurch erreichen, dass der Grad der extrakorporalen Unterstützung bei der Oxygenierung % ECLS_O2 einen Maximalwert für den Fluss des durch die ECLS-Einrichtung dem Patienten entnommenen Blutes festlegt. Dabei kann vorgesehen sein, dass der Maximalwert für den Fluss des durch die ECLS-Einrichtung dem Patienten entnommenen Blutes mit zunehmendem Grad der extrakorporalen Unterstützung bei der Oxygenierung % ECLS_O2 ansteigt. Diese Maßnahme kann anstelle des oben genannten Zusammenhangs zwischen maximalem positiven endexpiratorischen Druck und Grad der extrakorporalen Unterstützung bei der Oxygenierung herangezogen werden. Besonders effektiv ist es aber, diese Maßnahme zusätzlich heranzuziehen.

Zusätzlich oder alternativ kann man auch dem durch die ECLS-Einrichtung vorgegebenen Niveau des extrakorporalen Blutgasaustauschs einen Grad der extrakorporalen Unterstützung bei der Ventilation, d.h. bei der Abreicherung von CO2 aus dem Blut, zuordnen. Auch der Grad der extrakorporalen Unterstützung bei bei der Ventilation kann insbesondere ein relativer Wert sein, der sich auf den Anteil bezieht, den die extrakorporale Ventilation an der Abreicherung von CO2 aus dem Blut insgesamt hat, d.h. an der durch extrakorporale Ventilation und maschinelle Beatmung insgesamt bewirkten Abreicherung von CO2 aus dem Blut. Dieser Anteil soll im Folgenden auch als %ECLS_CO2 bezeichnet werden.

Der Grad der extrakorporalen Unterstützung bei der Ventilation %ECLS_CO2 kann unabhängig von dem Grad der extrakorporalen Unterstützung bei der Oxygenierung % ECLS_O2 sein.

Der Grad der extrakorporalen Unterstützung bei der Ventilation %ECLS_CO2 kann ein maximales Minutenvolumen (das Minutenvolumen ist definiert als Tidalvolumen multipliziert mit der Atemfrequenz) für die maschinelle Beatmung festlegen. Insbesondere kann das maximale Minutenvolumen mit abnehmendem Grad der extrakorporalen Unterstützung bei der Ventilation %ECLS_CO2 ansteigen. Zusätzlich oder alternativ kann der Grad der extrakorporalen Unterstützung bei der Ventilation %ECLS_CO2einen maximalen Atemwegsdruck (der Atemwegsdruck ist definiert als die Summe aus dem positiven endexpiratorischen Druck PEEP und dem während der Inspiration bzw. Expiration herrschenden Druck, üblicherweise wird der höchste Atemwegsdruck am Ende der Inspiration erreicht) für die maschinelle Beatmung festlegen. Insbesondere kann der maximale Atemwegsdruck mit abnehmendem Grad der extrakorporalen Unterstützung bei der Ventilation %ECLS_CO2 ansteigen. Als besonders geeignet erweist es sich, wenn der maximale Atemwegsdruck nicht nur vom Grad der extrakorporalen Unterstützung bei der Ventilation abgeleitet wird, sondern auch vom Grad der extrakorporalen Unterstützung bei der Oxytenierung, insbesondere vom jeweiligen größeren der beiden Grade. Beispielsweise kann zur Ableitung des maximalen Atemwegsdrucks bei der maschinellen Beatmung eine mit abnehmendem Grad des Maximalwerts aus extrakorporaler Unterstützung bei der Oxygenierung (%ECLS_O2) und extrakorporaler Unterstützung bei der Ventilation (%ECLS_CO2) ansteigende Beziehung zum maximalen Atemwegsdruck herangezogen werden.

Auf diese Weise wird dafür gesorgt, dass der maximale Atemwegsdruck und/oder das maximale Minutenvolumen - auch diese Größen sind bei vielen Beatmungsmodi der maschinellen Beatmung eine Rahmenbedingung für die Intensität der Beatmung - immer weiter ansteigt, je geringer der Grad der extrakorporalen Unterstützung bei der Ventilation %ECLS_CO2, ggf. unter zusätzlicher Berücksichtigung des Grads der extrakorporalen Unterstützung bei der Oxygenierung, wird. Damit steigt dementsprechend auch das Niveau der Unterstützung der Abreicherung von CO2 durch die maschinelle Beatmung im Vergleich zum Niveau der Unterstützung durch extrakorporale Ventilation. Die maschinelle Beatmung gewinnt mehr und mehr an Bedeutung je geringer der Grad der extrakorporalen Unterstützung bei der Ventilation wird. Es wird somit auch hierbei die Funktion der Lunge als (natürliches) Organ zur Abreicherung von CO2 aus dem Blut immer bedeutender, je geringer der Grad der extrakorporalen Unterstützung bei der Ventilation wird. Dem entspricht, dass die maschinelle Beatmung bei höheren maximalen Grenzwerten für den Atemwegsdruck und/oder das Minutenvolumen tendenziell eine effizientere Ausatmung bewirken kann, dies allerdings wiederum bei insgesamt höherer Belastung des Lungengewebes.

Auch für diesen Fall kann man eine Veränderung des Niveaus des extrakorporalen Blutgasaustauschs auch unmittelbar erreichen, wenn der Grad der extrakorporalen Unterstützung bei der Ventilation %ECLS_CO2 einen Maximalwert des Flusses von Oxygenierungsgas, das dem dem Blutkreislauf des Patienten entnommenen Blut durch die ECLS Einrichtung zugeführt wird, festlegt. Insbesondere kann der Maximalwert des Flusses von Oxygenierungsgas, das dem dem Blutkreislauf des Patienten entnommenen Blut durch die ECLS Einrichtung zugeführt wird, mit zunehmendem Grad der extrakorporalen Unterstützung bei der Ventilation %ECLS_CO2 ansteigen. Alternativ oder zusätzlich könnte man auch die Zusammensetzung des Oxygenierungsgases verändern. Auch diese Maßnahme kann anstelle der oben genannten Zusammenhänge zwischen maximalem Atemwegsdruck und/oder maximamalem Minutenvolumen einerseits und Grad der extrakorporalen Unterstützung bei der Ventilation andererseits herangezogen werden. Besonders effektiv ist es aber, diese Maßnahme zusätzlich heranzuziehen.

Ein in zuverlässiger Weise weitgehend - falls gewünscht sogar vollständig - automatisiert arbeitendes Beatmungsssystem wird in der Regel vorsehen, dass die ECLS-Einrichtung - bei einem eingestellten Wert für das Niveau des extrakorporalen Blutgasaustauschs - nach Ablauf einer vorbestimmten Zeit überprüft, ob bei dem eingestellten Wert für das Niveau des extrakorporalen Blutgasaustauschs durch die Einrichtung zur maschinellen Beatmung und die ECLS-Einrichtung gemeinsam ein vorbestimmter Zielzustand für den Blutgasaustausch erreicht wird.

Der vorbestimmte Zielzustand für den Blutgasaustausch kann beispielsweise durch eine Größe ausgedrückt sein, die für den Anteil von O2 im Blutkreislauf charakteristisch ist. Hierfür können im Prinzip alle gängigen Verfahren bzw. Größen herangezogen werden, mittels denen sich ein Anteil von O2 im Blut ausdrücken lässt. Insbesondere wird man gerne auf eine der folgenden Größen zurückgreifen: SpO2 (mittels Pulsoxymetrie bestimmter Sättigungswert von O2 im venösen Blut; hierfür stehen besonders einfach einsetzbare Fingerkuppensonden zur Verfügung), SaO2 (Sättigungswert von Sauerstoff im Blut, bestimmt mittels chemischer Analyse oder mittels optischer Verfahren) oder PaO2 (Partialdruck von O2 im But). Diese Werte können in der Einrichtung zur maschinellen Beatmung gemessen werden (z.B. durch Pulsoxymetrie) oder in der ECLS-Einrichtung gemessen werden (dies bietet sich zu Blutgasanalysen an, da ohnehin ein Strom vom Blut aus dem Gefäßsystem des Patienten abgezweigt wird).

Die Lage der Messsonden kann grundsätzlich stromabwärts oder stromaufwärts der Stelle liegen, an der Blutgasaustausch stattfindet.

In entsprechender Weise kann zusätzlich oder alternativ der vorbestimmte Zielzustand für den Blutgasaustausch durch eine Größe gekennzeichnet sein, die den Anteil von CO2 im Blutkreislauf kennzeichnet. Auch hierfür können sämtliche bekannte Verfahren bzw. Größen herangezogen werden, mittels denen sich ein Anteil bzw. eine Konzentration von CO2 im Blut ausdrücken lässt, z.B. Pa-CO2 (Partialdruck von CO2 im Blut), PetCO2 (Anteil von CO2 in der Atemluft, gemessen am Ende der Expirationsphase). Auch die Messung des Blutgasaustauschs hinsichtlich CO2 kann sowohl in der Einrichtung zur maschinellen Beatmung (z.G. PetCO2) oder in der ECLS-Einrichtung (z.B. PaCO2) erfolgen. Das oben hinsichtlich des Anteils von O2 Gesagte gilt entsprechend.

Eine besonders günstige Ausgestaltung sieht vor, dass die ECLS-Einrichtung bei Erreichen des vorbestimmten Zielzustands das Niveau des extrakorporalen Blutgasaustauschs verringert. Dies bedeutet, dass die ECLS-Einrichtung eine ihr innewonnende Tendenz hat, das Niveau des extrakorporalen Blutgasaustauschs - zugunsten des mittels maschineller Beatmung herbeigeführten Blutgasaustauschs - von einem Anfangsniveau ausgehend sukzessive immer weiter zu verringern. Damit ergibt sich ein dem Beatmungssystem inhärentes _{"}Weaning" vom extrakorporalen Blutgasaustausch: Der extrakorporale Blutgasaustausch wird tendenziell immer weiter verringert, solange sich die maschinelle Beatmung - für das gegebene Niveau des extrakorporalen Blutgasaustauschs - auf einen Zustand einstellen kann, bei dem ein vorgegebener Zielzustand erreicht ist. Wie bereits erläutert, ist der vorgegebenen Zielzustand insbesondere ein gewünschter Zustand hinsichtlich der erreichten Anreicherung des Blutes mit Sauerstoff und/oder hinsichtlich der erreichten Abreicherung von CO2 aus dem Blut.

Die gewünschte _{"}Weaning" Funktionalität lässt in besonders eleganter Weise dadurch erreichen, dass die ECLS-Einrichtung dann, wenn festgestellt wird, dass bei der Zielzustand erreichbar ist, insbesondere der vorgegebene Wert für die Konzentration von O2 im Blutkreislauf erreichbar ist, den Grad der extrakorporalen Unterstützung bei der Oxygenierung %ECLS_02, um einen ersten vorbestimmten Betrag verringert. Dadurch wird, wie oben erläutert, der Anteil der maschinellen Beatmung an der Unterstützung beim Blutgasaustausch, insbesondere an der Anreicherung des Blutes mit Sauerstoff, weiter erhöht auf Kosten des Anteils der extrakorporalen Unterstützung bei der Oxygenierung. Das Gesamtniveau der Unterstützung beim Blutgasaustausch, insbesondere bei der Oxygenierung, durch ECLS-Einrichtung und Einrichtung zur maschinellen Beatmung gemeinsam, braucht sich dabei nicht notwendigerweise zu verändern. Vielmehr wird es in vielen Fällen konstant bleiben, entsprechend der Tatsache, dass das insgesamt erforderliche Unterstützungsniveau sich nicht verändert hat. Was sich allerdings im Laufe der Zeit immer mehr verschiebt, ist der Anteil der maschinellen Beatmung an der insgesamt verabreichten Unterstützung. Zurückzuführen ist das beispielsweise darauf, dass die maschinelle Beatmung insgesamt im Rahmen immer lockerer Randbedingungen, etwa für den positiven endexpiratorischen Druck PEEP, stattfinden kann, weil der maximale endexpiratorische Druck immer größer wird je kleiner der Unterstützungsgrad durch extrakorporale Membranoxygenierung % ECLS_O2 wird. Auch einen Beitrag leistet die Tatsache, dass der maximale Fluss des dem Patienten entnommenen Blutes immer kleiner wird, je kleiner der Unterstützungsgrad durch extrakorporale Membranoxygenierung % ECLS_O2 wird.

Dementsprechend kann alternativ oder zusätzlich die ECLS-Einrichtung dann, wenn festgestellt wird, dass der Zielzustand erreichbar ist, insbesondere der vorgegebenen Wert für die Konzentration von CO2 im Blutkreislauf erreichbar ist, den Grad der extrakorporalen Unterstützung bei der Ventilation %ECLS_CO2, um einen zweiten vorbestimmten Betrag verringern. Das voranstehend mit Bezug auf den Grad der extrakorporalen Unterstützung bei der Oxidation %ECLS O2 Gesagte gilt in diesem Fall entsprechend: Der Anteil der maschinellen Beatmung an der Unterstützung beim Blutgasaustausch, insbesondere an der Abreicherung von Kohlendioxid aus dem Blut, wird immer weiter erhöht auf Kosten des Anteils der extrakorporalen Unterstützung bei der Ventilation. Das Gesamtniveau der Unterstützung beim Blutgasaustausch, insbesondere bei der Ventilation, durch ECLS-Einrichtung und Einrichtung zur maschinellen Beatmung gemeinsam, braucht sich dabei nicht notwendigerweise zu verändern. Vielmehr wird es in vielen Fällen konstant bleiben, entsprechend der Tatsache, dass das insgesamt erforderliche Unterstützungsniveau sich nicht verändert hat. Was sich allerdings im Laufe der Zeit immer mehr verschiebt, ist der Anteil der maschinellen Beatmung an der insgesamt verabreichten Unterstützung. Die maschinelle Beatmung kann insgesamt im Rahmen immer lockerer Randbedingungen, etwa für den maximalen Atemwegsdruck und/oder das maximale Tidalvolumen, stattfinden, weil der maximalen Atemwegsdruck und/oder das maximale Tidalvolumen immer größer wird, je kleiner der Unterstützungsgrad durch extrakorporale Ventilation %ECLS_CO2 wird. Hierzu trägt auch bei, wenn der maximale Fluss des dem Patienten entnommen Blut zugeführten Oxydierungsgases immer kleiner wird, je kleiner der Unterstützungsgrad durch extrakorporale Ventilation %ECLS_CO2 wird.

Wird der vorbestimmte Zielwert sogar überschritten, kann vorgesehen sein, dass die ECLS-Einrichtung das Niveau der extrakorporalen Oxygenierung verringert. Insbesondere kann vorgesehen sein, dass die ECLS-Einrichtung bei Überschreiten des vorgegebenen Werts der Konzentration von O2 im Blutkreislauf den Fluss des durch die ECLS Einrichtung aus dem Blutkreislauf des Patienten entnommenen Blutes verringert und/oder den Grad der extrakorporalen Unterstützung bei der Oxygenierung verringert. Insbesondere kann vorgesehen sein, dass die ECLS-Einrichtung bei Überschreiten des vorgegebenen Werts für die Konzentration von CO2 im Blutkreislauf den Fluss von Oxygenierungsgas, das dem dem Blutkreislauf des Patienten entnommenen Blut durch die ECLS Einrichtung zugeführt wird, verringert und/oder den Grad der extrakorporalen Unterstützung bei der Ventilation %ECMO_CO2 verringert. Die Verringerung des Grads der extrakorporalen Unterstützung bei der Oxygenierung bzw. des Grads der extrakorporalen Unterstützung bei der Ventilation erfolgt dabei insbesondere um einen größeren Betrag als für den Fall, dass der jeweilige vorbestimmte Zielwert zwar erreicht, aber nicht überschritten wird.

Wird der vorbestimmte Zielwert nicht erreicht, kann ein Stehenbleiben bzw. eine gewisse Umkehr des Prozesses vorgesehen sein. Diese kann dadurch erreicht werden, dass die ECLS-Einrichtung das Niveau der extrakorporalen Oxygenierung bzw. das Niveau der extrakorporalen Ventilation jeweils um einen vorbestimmten Betrag erhöht. In diesem Szenario kann auch Gefahrensituationen Rechnung getragen werden, indem eine Erhöhung des Niveaus der extrakorporalen Oxygenierung bzw. des Niveaus der extrakorporalen Ventilation um einen deutlich größeren Betrag vorgesehen wird, wenn der jeweilige Zielwert ganz deutlich verfehlt wird und/oder keinerlei Tendenz zur Annäherung an den jeweiligen Zielwert feststellbar ist.

Ein sich weitgehend selbstständig einem Zustand mit möglichst großem Anteil an maschineller Beatmung, bei so hohem Anteil an extrakorporalem Blutgasaustausch wie erforderlich, annäherndes Beatmungssystem lässt sich insbesondere dadurch realisieren, dass die ECLS-Einrichtung in wiederkehrenden Zeitabständen überprüft, ob bei dem jeweils eingestellten Wert für das Niveau des extrakorporalen Blutgasaustauschs durch die Einrichtung zur maschinellen Beatmung und die ECLS-Einrichtung gemeinsam ein vorbestimmter Zielwert für den Blutgasaustausch erreichbar ist. Diese Überprüfung kann in der vorangehend umrissenen Weise erfolgen und zu den vorangehend ausgeführten Konsequenzen führen.

Der Zeitabstand für die Überprüfung durch die ECLS-Einrichtung wird dabei nach der Regel _{"}so klein wie möglich, so groß wie erforderlich" zu wählen sein. Man kann dabei ausnutzen, dass die Einrichtung zur maschinellen Beatmung in der Lage ist, sich innerhalb sehr kurzer Zeitskalen auf veränderte Umstände einzustellen. Extrakorporaler Blutgasaustausch verlangt dagegen einen deutlich stärkeren Eingriff, was auch bedeutet, dass starke Rückwirkungen auf den Patienten zu erwarten sind. Deswegen wird vorgeschlagen, die Parameter für den extrakorporalen Blutgasaustausch so langsam und so kontinuierlich wie möglich anzupassen. Dem kann man dadurch Rechnung tragen, dass der Zeitabstand für die Überprüfung durch die ECLS-Einrichtung deutlich größer gewählt wird als die Zeitkonstante der Einrichtung zur maschinellen Beatmung, d.h. der Zeit, die die Einrichtung zur maschinellen Beatmung im Mittel braucht, um sich auf eine Veränderung der vorgegebenen Parameter einzustellen. Die vorgeschlagene Kombination der Zusammenwirkung von ECMO als den Therapieverlauf im großen Zügen vorgebendes Teilsystem mit maschineller Beatmung als sich jeweils darauf einstellendes Teilsystem kommt diesem Ansatz entgegen, weil es von Haus aus erlaubt, den zeitlichen Abstand der Überprüfung durch die ECLS-Einrichtung eher groß zu wählen, auf alle Fälle sehr viel größer als die Zeitkonstante der maschinellen Beatmung. Beispielsweise kann die Einrichtung zur maschinellen Beatmung so ausgelegt sein, dass sie mit jedem Atemzug eine neue Einstellung vornimmt, also atemzugweise regelt.

Grundsätzlich kann man so vorgehen, dass die ECLS-Einrichtung von einem voreingestellten Startwert für das Niveau der extrakorporalen Oxygenierung/Ventilation ausgeht und dann im Zusammenspiel mit der Einrichtung zur maschinellen Beatmung weitgehend selbstständig, d.h. ohne obligatorische manuelle Eingriffe, zu einem Zustand gelangt, in dem beide Teilsysteme weitgehend optimal arbeiten. Dieser Zustand kann sich dann auch im Verlauf der Therapie verändern, wenn beispielsweise Änderungen des Zustands des Patienten eintreten, die eine Anpassung der maschinellen Beatmung und/oder des extrakorporalen Blutgasaustauschs erforderlich machen. Besonders günstig ist es, wenn der Startwert einem maximalen Niveau für das Niveau der extrakorporalen Oxygenierung/Ventilation entspricht. Man geht dann von einem Zustand aus, bei dem der Blutgasaustausch praktisch vollständig durch die ECLS-Einrichtung und ohne die Einrichtung zur maschinellen Beatmung erfolgt. Von diesem Zustand ausgehend, passt das Beatmungssystem das Niveau von extrakorporalem Blutgasaustausch und maschineller Beatmung Schritt um Schritt an, wobei der Anteil des extrakorporalen Blutgasaustauschs immer weiter zurückgeführt wird und dementsprechend der Anteil der maschinellen Beatmung Schritt um Schritt hochgefahren wird.

Es ist günstig, wenn das Beatmungssystem den Startwert als Bezugsgröße für die in weiteren Verlauf stattfindende(n) Verringerung(en) bzw. Erhöhung(en) des Niveaus der extrakorporalen Oxygenierung/Ventilation nimmt.

Die vorliegende Offenbarung betrifft darüber hinaus auch ein Verfahren zum koordinierten Betreiben einer Einrichtung zur maschinellen Beatmung der Lunge eines Patienten, und einer ECLS-Einrichtung zum extrakorporalen Blutgasaustausch des Blutes des Patienten, bei dem maschinelle Atmungsunterstützung durch die Einrichtung zur maschinellen Beatmung einerseits und ein extrakorporaler Blutgasaustausch durch die ECLS-Einrichtung andererseits in koordinierter Weise automatisiert durchgeführt werden, um den Gasaustausch im Blutkreislauf des Patienten zu unterstützen. Bei diesem Verfahren gibt die ECLS-Einrichtung ein Niveau des extrakorporalen Blutgasaustauschs vor und stellt sich die Einrichtung zur maschinellen Beatmung anhand des von der ECLS-Einrichtung vorgegebenen Niveaus des extrakorporalen Blutgasaustauschs automatisiert auf ein Niveau der maschinellen Atmungsunterstützung ein. Ein derartiges Verfahren kann in bevorzugten Weiterbildung ausgestaltet sein wie in den vorangehenden Absätzen beschrieben.

Die Erfindung wird nachfolgend anhand von Ausführungsbeispielen unter Bezugnahme auf Zeichnungen näher erläutert. Es zeigt:
- Fig. 1:: Eine schematische und stark vereinfachte Ansicht eines erfindungsgemäßen Beatmungssystems mit als Überdruckbeatmungseinrichtung ausgebildeter Einrichtung zur maschinellen Beatmung und ECLS-Einrichtung.
- Fig. 2:: Eine schematische und stark vereinfachte Ansicht gemäß Figur 1 eines weiteren erfindungsgemäßen Beatmungssystems mit als Überdruckbeatmungseinrichtung ausgebildeter Einrichtung zur maschinellen Beatmung und ECLS-Einrichtung.
- Fig. 3:: Ein Flussdiagramm, das den Ablauf der koordinierten Zusammenwirkung zwischen Einrichtung zur maschinellen Beatmung und ECLS-Einrichtung in einem Beispiel verdeutlicht.
- Fig. 4:: Ein Flussdiagramm, das weitere Details der in dem Oxygenierungs-Modul gemäß Fig. 3 ablaufenden Prozeduren verdeutlicht.
- Fig. 5:: Ein Flussdiagramm, das weitere Details der in dem Ventilations-Modul gemäß Fig. 3 ablaufenden Prozeduren verdeutlicht.
- Fig. 6:: Ein Diagramm, das qualitativ den Zusammenhang zwischen dem Grad der extrakorporalen Unterstützung bei der Oxygenierung % ECLS_O2 und dem maximalen positiven endexpiratorischen Druck PEEPmax zeigt.
- Fig. 7:: Ein Diagramm, das qualitativ den Zusammenhang zwischen dem Grad der extrakorporalen Unterstützung bei der Oxygenierung % ECLS_O2 und dem in der ECLS-Einrichtung einstellbaren maximalen Pumpenfluss zeigt.
- Fig. 8:: Ein Diagramm, das qualitativ den Zusammenhang zwischen dem Grad der extrakorporalen Unterstützung bei der Ventilation %ECLS_CO2 und dem maximalen Minutenvolumen bei der Überdruckbeatmung zeigt.
- Fig. 9:: Ein Diagramm, das qualitativ den Zusammenhang zwischen dem Maximum aus Grad der extrakorporalen Unterstützung bei der Oxygenierung %ECLS O2 und Grad der extrakorporalen Unterstützung bei der Ventilation %ECLS_CO2 einerseits sowie dem maximalen Atemwegsdruck (Pinsp+PEEP)max bei der Überdruckbeatmung zeigt.
- Fig. 10:: Ein Diagramm, das qualitativ den Zusammenhang zwischen dem Grad der extrakorporalen Unterstützung bei der Ventilation %ECLS_CO2 und dem in der ECLS-Einrichtung einstellbaren maximalen Fluss von Oxygenierungsgas zeigt.
- Fig. 11:: Ein Diagramm, das in qualitativer Weise andeutet, welche Werte für den mittels Pulsoxymetrie gemessenen Sauerstoffsättigungswert SpO2 bei jeweils herrschendem positiven endexpiratorischem Druck PEEP als zu hoch, zu niedrig, normal oder gar als Notfall angesehen werden und in der Abfrage gem. Fig. 4 zu entsprechenden Veränderungen des Parameters %ECLS_O2 führen.
- Fig. 12:: Ein Diagramm, das in qualitativer Weise andeutet, welche Werte für den im arteriellen Blut gemessene Kohlendioxidkonzentration PaCO2 bei jeweils herrschendem maximalen Atemwegsdruck (Pinsp + PEEP) als zu hoch, zu niedrig, oder normal angesehen werden und in der Abfrage gem. Fig. 5 zu entsprechenden Veränderungen des Parameters %ECLS_CO2 führen.

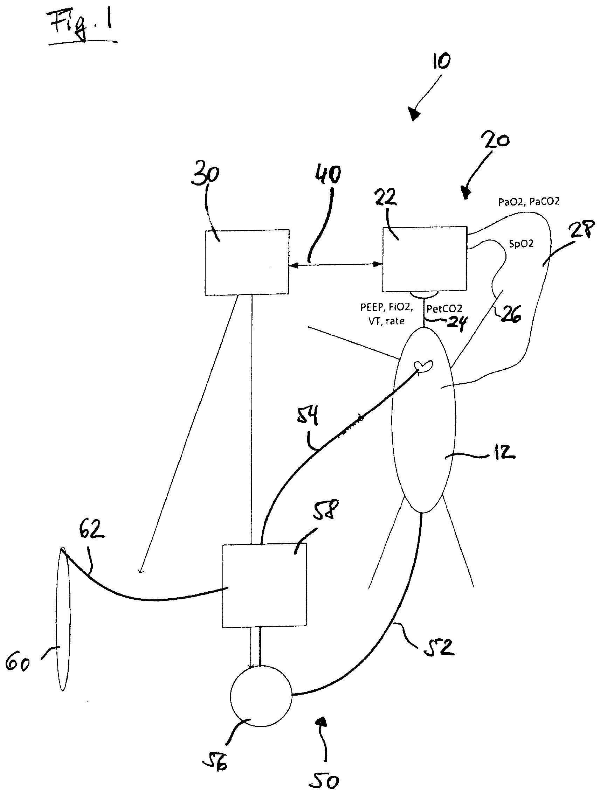

Fig. 1 zeigt in schematischer und stark vereinfachter Ansicht ein erfindungsgemäßen Beatmungssystem 10 mit als Überdruckbeatmungseinrichtung ausgebildeter Einrichtung zur maschinellen Beatmung 20 und ELCS-Einrichtung 50. Die Beatmungseinrichtung 20 umfasst ein Beatmungsgerät 22, das in Fig. 1 nur schematisch angedeutet ist. Das Beatmungsgerät 22 ist über ein nicht näher dargestelltes Leitungssystem 24 mit dem Luftweg eines Patienten (in Fig. 1 schematisch angedeutet und mit 12 bezeichnet) verbunden. Über das Leitungssystem 22 beaufschlagt das Beatmungsgerät 22 den Atemweg des Patienten 12 mit Luft unter Überdruck während der Inspirationsphasen und leitet Luft aus dem Atemweg des Patienten während der Expirationsphasen. Das Leitungssystem 22 wird während der Beatmung dauerhaft mit einem positiven endexpiratorischen Druck PEEP beaufschlagt. Zu diesem Druck addiert sich während der Inspirationsphasen ein ebenfalls vom Beatmungsgerät beaufschlagter Inspirationsdruck Pinsp (der sich innerhalb des Inspirationszyklus in der Regel verändert). Während der Expirationsphasen beaufschlagt das Beatmungsgerät in der Regel nur den positiven endexpiratorischen Druck PEEP, gegen den sich das Lungengewebe entspannt.

Das Beatmungseinrichtung 20 ist ferner mit Sensorik ausgestattet, um für die Beatmung wesentliche Größen zu erfassen. Im Leitungssystem 24 werden beispielsweise die folgenden zum Teil in Fig. 1 angedeuteten Größen erfasst: Inspirationsdruck Pinsp, Expirationsdruck Pexp, positiver endexpiratorischer Druck PEEP, Tidalvolumen VT (d.h. Atemgasvolumen, das während eines lnspirationszyklus in die Lunge appliziert wird), Flussmenge des applizierten Atemgases, Partialdruck von CO2 im Atemgas (insbesondere während der Expiration). Die Beatmungseinrichtung 20 verfügt außerdem noch über Sensorik zur Bestimmung der Sauerstoffsättigung im Blut des Patienten, in Fig. 1 mit dem Bezugszeichen 26 angedeutet. Diese kann als Pulsoxymetrie-Sensorik ausgebildet sein (z.B. mit Fingerkuppen-Sensor) zur subkutanen Bestimmung der arteriellen Sauerstoffsättigung SpO2. Bei der in Fig. 1 gezeigten Ausführungsform verfügt die Beatmungseinrichtung 20 weiterhin über Sensorik zur Analyse von Blutgasen, insbesondere zur Bestimmung der arteriellen Konzentration von Sauerstoff im Blut PaO2 und zur Bestimmung der arteriellen Konzentration von Kohlnendioxid im Blut PaCO2in wiederkehrenden Abständen, wie in Fig. schematisch mit 28 angedeutet. Anstelle der Konzentrationen PaO2 und PaCO2von Sauerstoff bzw. Kohlendioxid können auch die entsprechenden Sättigungen im Hämoglobin SaO2 und SaCO2 bestimmt werden.

Der Beatmungseinrichtung 20 ist eine Steuerung zugeordnet, die dafür ausgebildet ist, alle Abläufe der Beatmungseinrichtung weitgehend automatisiert zu steuern bzw. zu regeln. Diese Steuerung kann in das Beatmungsgerät 22 integriert sein, sie kann aber auch teilweise oder sogar ganz als externes Steuergerät ausgebildet sein. Die Steuerung für die Beatmungseinrichtung 20 besitzt die üblichen Schnittstellen zur Kommunikation mit Bedienpersonal, insbesondere zur Anzeige des Beatmungszustands des Patienten und zur Eingabe von Steuerbefehlen. Im Grundsatz ist die Steuerung so ausgebildet, dass die Beatmungseinrichtung 20 weitgehend selbstständig und ohne manuelle Eingriffe geeignete Beatmungsmodi auswählt bzw. im Rahmen eines vorgewählten Beatmungsmodus die Beatmungsparameter auf jeweils optimale Werte einstellt und im Sinne geschlossener Regelkreise auch die Beatmungsparameter selbständig überwacht und ggf. nachstellt, so dass ein gewünschter Beatmungszustand möglichst beibehalten werden kann.

Beatmungseinrichtungen dieser Art sind beispielsweise solche, die über weitgehend automatisierte Beatmungsmodi verfügen, etwa den unter der Bezeichnung _{"}Adaptive Support Ventilation" bekannten Beatmungsmodus, über den Beatmungseinrichtungen der Anmelderin verfügen.

Zur zusätzlichen Unterstützung des Blutgasaustauschs ist bei dem Beatmungssystem 10 in Fig. 1 eine ECLS-Einrichtung (ECLS = extracorporal lung support) vorgesehen, die allgemein mit dem Bezugszeichen 50 bezeichnet ist. Im Gegensatz zur Beatmungseinrichtung 20, die an den Atemweg des Patienten angeschlossen ist und über diesen die Lunge mit Atemgas beaufschlagt, dient die ECLS-Einrichtung 50 dazu, den Austausch von Blutgasen unmittelbar zu unterstützen, also die Funktion der Lunge teilweise oder sogar ganz zu ersetzen. Die ECLS-Einrichtung 50 ist daher nicht an die Lunge angekoppelt, sondern unmittelbar an den Blutkreislauf des Patienten. Die ECLS-Einrichtung 50 verfügt über eine erste Leitung 52, über die Blut aus dem venösen System des Patienten entnommen wird und in einen extrakorporalen Blutkreislauf der ECLS-Einrichtung 50 gelangt. Der extrakorporale Blutkreislauf wird angetrieben von einer ECLS-Pumpe 56, welche das aus dem venösen System entnommene Blut zu einem Oxygenator 58 fördert und dann über eine weitere Leitung 54 zurück in den Blutkreislauf des Patienten bringt. Im Falle veno-venöser extrakorporaler Blutgasaustauschunterstützung fördert die weitere Leitung 54 das mit Sauerstoff angereicherte und von CO2 abgereicherte Blut zurück in das venöse System des Patienten. Auch denkbar sind Formen von veno-arterieller extrakorporaler Blutgasaustauschunterstützung, bei denen die weitere Leitung 54 das mit Sauerstoff angereicherte und von CO2 abgereicherte Blut zurück in das arterielle System des Patienten stromabwärts des Herzens fördert, um neben der pulmonalen Funktion auch die kardiale Funktion des Patienten zu unterstützen. Die ECLS-Einrichtung 50 ist hinsichtlich ihrer Funktionsweise dann sehr ähnlich zu einer Herz-Lungen-Maschine.

Im Oxygenator 58 wird das venöse Blut von CO2 abgereichert und mit O2 angereichert. Hierzu wird dem Oxygenator 58 ein Oxygenierungsgas 62 zugeführten, welches im Oxygenator 58 mit dem venösen Blut interagiert um CO2 vom Hämoglobin aufzunehmen und das dabei frei werdende Hämoglobin mit O2 zu belegen. Der Oxygenator 58 übernimmt also im Wesentlichen die Funktion der Alveolen in der Lunge. Derartige Oxygenatoren 58 sind beispielsweise bei Herz-Lungen-Maschinen bekannt. Bei Ausführung als Membranoxygenator wird eine semipermeable Membran zum Austausch der Blutgase O2 und CO2 eingesetzt und die durch die ECLS-Einrichtung geleistete Unterstützung beim Blutgasaustausch ist unter der Bezeichnung ECMO (extrakorporale Membranoxygenierung) bekannt. Auch andere Oxygenator-Bauweisen sind bekannt und können grundsätzlich eingesetzt werden. Das Oxygenierungsgas 62 stammt aus einer Konditionierungseinheit 60 und ist grundsätzlich ein mit O2 angereichertes Gasgemisch, in manchen Fällen auch reines O2. Das Ausmaß der gewünschten Oxygenierung bzw. Ventilation kann durch die Partialdrücke von O2 bzw. CO2 im Oxygenierungsgas 62 eingestellt werden. Hierzu kann des Oxygenierungsgas in geeigneter Weise konditioniert sein, falls gewünscht kann das auch in Abhängigkeit der Zusammensetzung von Blutgasen im venösen System, aus dem der extrakorporale Blutgasstrom entnommen wird, oder im arteriellen System geschehen.

Die grundsätzlichen Parameter zur Steuerung des Niveaus der Blutgasaustauschunterstützung durch die ECLS-Einrichtung 50 sind der Fluss des dem Körper entnommenen und durch den extrakorporalen Kreislauf gepumpten Blutes sowie der Fluss des dem Oxygenator zugeführten Oxygenierungsgases. Der Fluss des dem Körper entnommenen und durch den extrakorporalen Kreislauf gepumpten Blutes lässt sich anhand des von der Pumpe 56 erzeugten Pumpenflusses recht leicht erfassen und einstellen. Auch der Fluss des Oxygenierungsgases lässt mittels geeigneter Durchflusssensoren bzw. -regler leicht erfassen und einstellen.

Daneben besteht noch die Möglichkeit, die Zusammensetzung des Oxygenierungsgases geeignet zu beeinflussen wie oben beschrieben. Beispielsweise kann man dem Oxygenierungsgas durchaus eine gewisse Menge CO2 beimengen, um negative physiologische Auswirkungen einer zu starken Abreicherung von CO2 zu unterdrücken.

Zur Steuerung der ECLS-Parameter dient eine ECLS-Steuerung, die in Fig. 1 als separates Steuergerät angedeutet und mit dem Bezugszeichen 30 versehen ist. Die ECLS-Steuerung 30 erfasst die angesprochenen Daten hinsichtlich des Flusses des dem Körper entnommenen und durch den extrakorporalen Kreislauf gepumpten Blutes sowie des Flusses des dem Oxygenator zugeführten Oxygenierungsgases sowie auch hinsichtlich der Zusammensetzung des Oxygenierungsgases. In Antwort auf diese Daten gibt die ECLS-Steuerung geeignete Steuerbefehle an die ECLS-Pumpe 56, den Oxygenator 58, sowie den Oxygenierungsgaserzeuger 60 und diesen zugeordnete Stellglieder ab, um die jeweils gewünschten Flüsse und Zusammensetzungen einzustellen, in der Regel über entsprechende geschlossene Regelkreise (z.B. Pl-Regelkreise). Dies soll in Fig. durch die jeweiligen Pfeile angedeutet sein.

Die der Beatmungseinrichtung zugeordnete Steuerung und die der ECLS-Einrichtung zugeordnete Steuerung arbeiten grundsätzlich unabhängig voneinander. Allerdings tauschen sie über eine in Fig. 1 mit 40 bezeichnete Verbindung Daten miteinander aus, um eine koordinierte Betriebsweise der Beatmungseinrichtung 20 einerseits und der ECLS-Einrichtung 50 andererseits zu ermöglichen. Diese soll nachfolgend noch näher beschrieben werden.

An dieser Stelle soll allerdings bereits darauf hingewiesen sein, dass eine physikalische Trennung von der ECLS-Einrichtung 50 zugeordneter Steuerung 30 und der Beatmungseinrichtung 20 zugeordneter Steuerung nicht unbedingt erforderlich ist. Es ist durchaus denkbar, beide Steuerungen physikalisch in einer Einheit bzw. einem Modul zusammenzufassen. Eine solche Einheit bzw. Modul kann als selbstständige Einheit neben der Beatmungseinrichtung 20 und der ECLS-Einrichtung 50 vorgesehen sein, sie kann aber auch vollständig in eine dieser Einrichtungen integriert sein, z. B. in das in Fig. 1 gezeigte Beatmungsgerät 22.

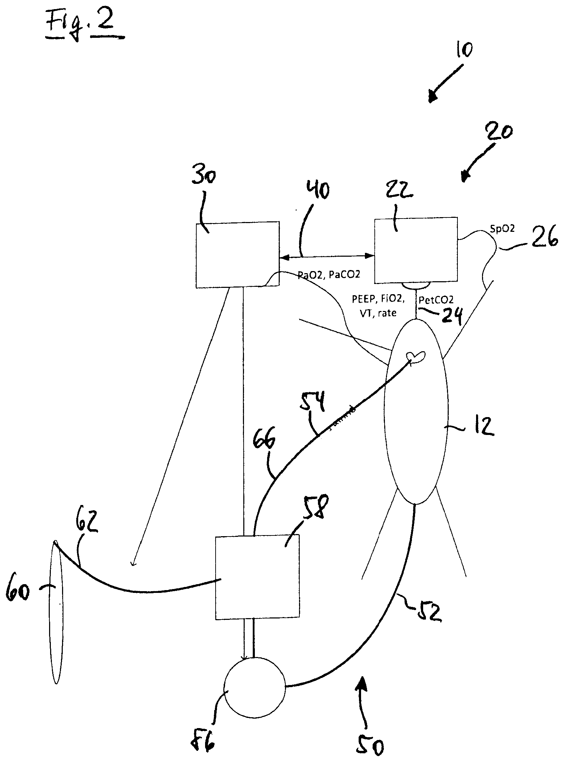

Fig. 2 zeigt in schematischer und stark vereinfachter Ansicht gemäß Fig.1 ein weiteres erfindungsgemäßes Beatmungssystem 10 mit Beatmungseinrichtung 20 und ELCS-Einrichtung 50. Die in Fig. 2 verwendeten Bezugszeichen entsprechen denen aus Fig. 1, soweit dieselben oder entsprechende Komponenten vorhanden sind. Insoweit wird auf die Beschreibung dieser Komponenten mit Bezug zu Fig. 1 verwiesen, die entsprechend gilt. Das Beatmungssystem gem. Fig. 2 unterscheidet sich von dem gem. Fig. 1 nur dadurch, dass die Sensorik zur Bestimmung der Konzentration bzw. Sättigung von O2 und CO2 (in der Form PaO2 bzw. PaCO2oder in der Form SaO2 bzw. SaCO2) im Blutkreislauf des Patienten nun nicht mehr der Beatmungseinrichtung 20 zugeordnet ist, sondern der ECLS-Einrichtung 50 zugeordnet ist. Wie durch das Bezugszeichen 66 angedeutet, findet die Bestimmung von PaO2/PaCO2 (oder SaO2/SaCO2) im extrakorporalen Blutkreislauf statt. Dies bietet sich vor allem deswegen an, weil im extrakorporalen Blutkreislauf derartige Sensorik gut unterzubringen ist und man zum Betrieb der ECLS-Einrichtung 50 in aller Regel ohnehin derartige Informationen braucht. Über die Schnittstelle 40 stehen die im ECLS-Blutkreislauf erfassten Daten dann auch für die Beatmungseinrichtung 20 zur Verfügung.

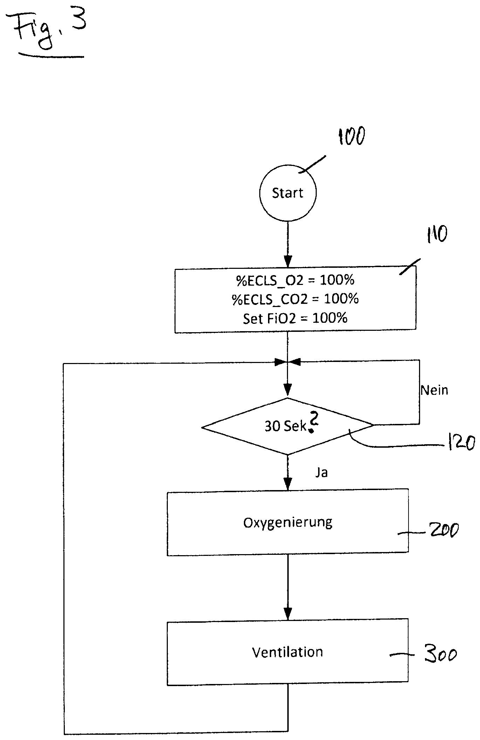

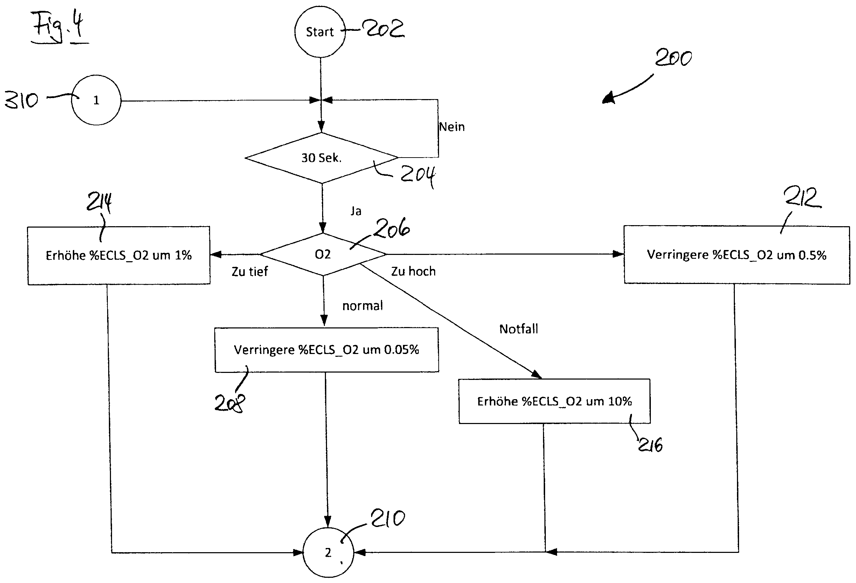

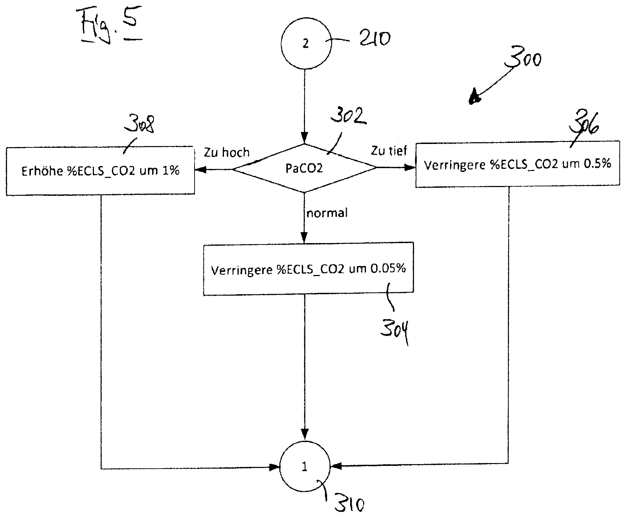

Figur 3 zeigt in Form eines Flussdiagramms den grundsätzlichen Ablauf der koordinierten Zusammenwirkung zwischen Beatmungseinrichtung 20 und ECLS-Einrichtung 50 in einem Beispiel. Das Flussdiagramm gem. Fig.3, wie auch die nachfolgend noch zu besprechenden Flussdiagramme gem. Fig. 4 und 5 beschränken sich dabei auf eine Darstellung der hinsichtlich der koordinierten Zusammenwirkung von Beatmungseinrichtung 20 und ECLS-Einrichtung 50 relevanten Schritte und Parameter ohne Anspruch auf Vollständigkeit hinsichtlich des Ablaufs der dargestellten Prozeduren insgesamt.

Nach Start des Ablaufs werden zunächst Startwerte für einige Parameter festgelegt, die hinsichtlich der koordinierten Zusammenwirkung von Beatmungseinrichtung 20 und ECLS-Einrichtung 50 Bedeutung haben. Dies sind vor allem die Parameter Grad der exkorporalen Unterstützung bei der Oxygenierung %ECLS O2 und Grad der exkorporalen Unterstützung bei der Ventilation %ECLS_CO2. Beide Größen werden gemäß dem dargestellten Ausführungsbeispiel als relative Größen ausgedrückt, die jeweils anzeigen sollen, welchen Anteil die durch die ECLS-Einrichtung 50 geleistete exkorporale Unterstützung beim Blutgasaustausch (d.h. bei Oxygenierung bzw. Ventilation) an der gesamten Unterstützung beim Blutgasaustausch hat. Dabei werden Oxygenierung und Ventilation getrennt betrachtet und durch je einen eigenen Grad an extrakorporaler Unterstützung % ECLS_O2 bzw. %ECLS_CO2 ausgedrückt. Sowohl dem Parameter %ECLS O2 als auch dem Parameter %ECLS_CO2 wird ein Startwert zugeordnet. Dieser kann manuell vorgegeben werden. Man wird sich bei Wahl der Startwerte im Allgemeinen am Zustand des Patienten orientieren und einer Abschätzung folgen, in welchem Anteil zusätzlicher exkorporaler Blutgasaustausch erforderlich sein dürfte, um in Zusammenwirkung mit der Überdruckbeatmung zu einem vernünftigen Gesamtzustand des Patienten zu kommen. Bei dieser Abschätzung wird empfohlen, "konservativ" vorzugehen, d.h. den Grad der extrakorporalen Unterstützung an der Oxygenierung bzw. an der Ventilation auf keinen Fall zu unterschätzen, sondern im Zweifelsfall die Startwerte für % ECLS_O2 und %ECLS_CO2 lieber zu hoch zu wählen. In dem gezeigten Beispiel werden Startwerte von jeweils 100% für % ECLS_O2 und %ECLS_CO2 gewählt, was bedeutet, dass die Unterstützung beim Blutgasaustausch am Anfang vollständig durch extrakorporalen Blutgasaustausch geleistet wird, die Überdruckbeatmung also gar keinen Beitrag leistet.

Daneben wird auch ein Startwert für den Anteil von Sauerstoff im Atemgas FiO2 festgelegt, das dem Atemweg durch die Beatmungseinrichtung 20 zugeführt wird. In dem in Fig. 3 gezeigten Beispiel wird ein Startwert von FiO2 = 100 % festgelegt, was bedeutet, dass die Beatmungseinrichtung dem Patienten reinen Sauerstoff zuführt, sobald sie neben der ECLS-Einrichtung 50 zur Unterstützung des Blutgasaustauschs beiträgt. Der in diesem Schritt festgelegte Wert für FiO2 wird sich allerdings sehr schnell ändern, weil die Beatmungseinrichtung 20 so eingerichtet ist, dass sie immer einen bestmöglichen Beatmungsmodus auswählt und für den ausgewählten Beatmungsmodus die Beatmungsparameter so einstellt, dass sich ein möglichst guter Beatmungszustand einstellt. Dabei wird die Beatmungseinrichtung 20 in der Regel den Wert von FiO2 verändern, insbesondere auf einen Wert deutlich kleiner als 100%.

Nach Einstellung der Startwerte wartet die Prozedur ab, ob eine vorbestimmte Zeit vergangen ist (Schritt 120). Diese vorbestimmte Zeit bestimmt die Wiederholrate der Neueinstellung der Parameter für die ECLS-Einrichtung 50. Sie sollte deutlich größer gewählt werden als die Zeitkonstante der Beatmungseinrichtung 20, d. h. die Zeit, die die Beatmungseinrichtung 20 im Mittel braucht, um sich auf einen neuen Zustand einzustellen. Im vorliegenden Beispiel beträgt diese Zeit 30s.

Sobald die vorbestimmte Zeit abgelaufen ist, wird eine Prozedur zur Einstellung relevanter Parameter hinsichtlich der Oxygenierung durchlaufen (Schritt 200) gefolgt von einer Prozedur zur Einstellung relevanter Parameter hinsichtlich der Ventilation (Schritt 300).

Dieser Ablauf wiederholt sich dann immer wieder, d.h. es wird wieder die vorbestimmte Zeit abgewartet (Schritt 120), dann die Oxygenierung durchlaufen (Schritt 200), gefolgt von der Ventilation (Schritt 300), usw.

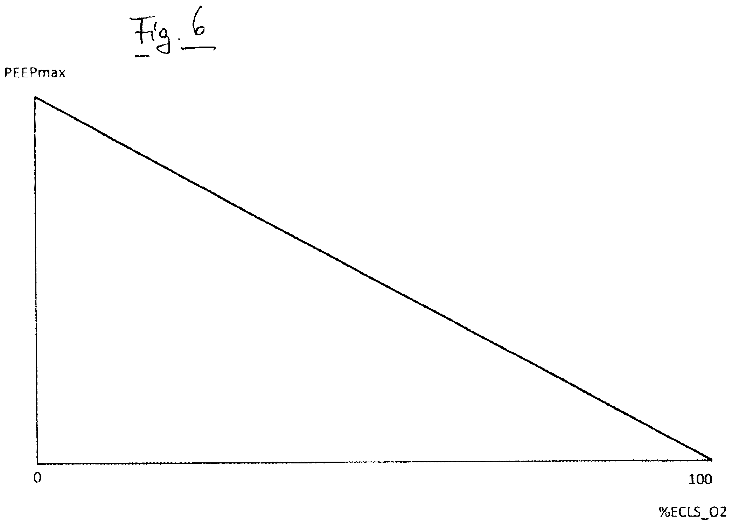

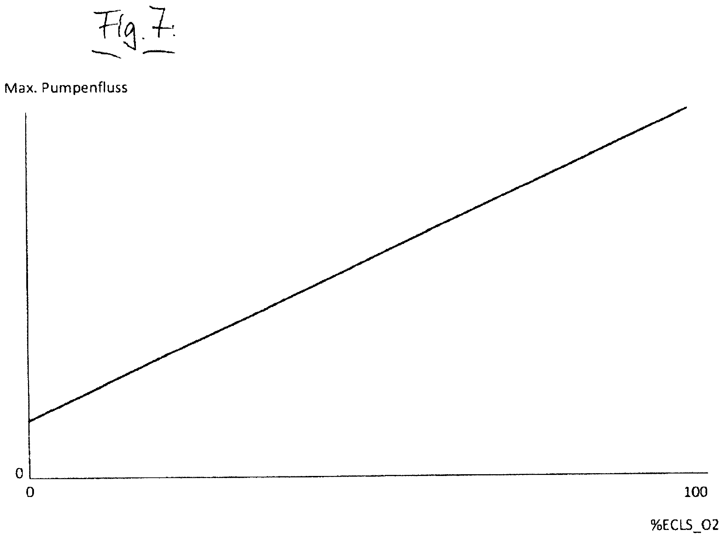

Fig. 4 zeigt ein Flussdiagramm, welches die in dem Oxygenierungs-Modul 200 gemäß Fig. 3 ablaufenden Prozeduren etwas detaillierter verdeutlicht. Nach Start in Schritt 202 wird zunächst eine vorbestimmte Zeit abgewartet (Schritt 204), die in dem gewählten Beispiel 30s beträgt. Nach Ablauf dieser Zeit wird in Schritt 206 überprüft, ob die Oxygenierung des Blutkreislaufs ausreichend ist. Dies kann anhand eines oder mehrerer der bereits geschilderten Sensoren erfolgen, etwa durch Bestimmung des Sauerstoffsättigungswerts SpO2 mittels Pulsoxymetrie, oder durch eine laufend erfolgende Analyse der Blutgaswerte mit Bestimmung der Sauerstoffkonzentration im Blut PaO2. Falls gewünscht kann auch die mittels Pulsoxymetrie bestimmte Sauerstoffsättigung SpO2 durch nur sporadisch mittels Blutgasanalyse ermittelte Werte für PaO2 oder SaO2 ergänzt werden. Ergibt die Bestimmung in Schritt 206, dass die Sauerstoffkonzentration bzw. Sauerstoffsättigung in einem erwünschten Bereich liegt, dann wird in Schritt 210 der Parameter %ECLS_O2, der den Grad der extrakorporalen Unterstützung bei der Oxygenierung ausdrückt, um einen vorbestimmten ersten Betrag verringert, in dem gezeigten Beispiel um 0,05%. Ab diesem Zeitpunkt verändern sich relativen Anteile der Unterstützung beim Blutgasaustausch zwischen extrakorporaler Blutgasaustauschunterstützung mittels ECLS-Einrichtung 50 und Unterstützung mittels Überdruckbeatmung. Dies lässt sich anhand des Schaubilder in Fig. 6 und 7 ablesen: Dem in Fig. 6 gezeigten Diagramm, das qualitativ den Zusammenhang zwischen dem Grad der extrakorporalen Unterstützung bei der Oxygenierung % ECLS_O2 und dem maximalen positiven endexpiratorischen Druck PEEPmax zeigt, ist zu entnehmen, dass die Verringerung des Parameters % ECLS_O2 um den ersten vorbestimmten Betrag einerseits den für die Beatmungseinrichtung 20 vorgegebenen maximalen positiven endexpiratorischen Druck PEEPmax erhöht. Andererseits entnimmt man dem Diagramm gem. Fig. 7, das qualitativ den Zusammenhang zwischen dem Grad der extrakorporalen Unterstützung bei der Oxygenierung % ECLS_O2 und dem in der ECLS-Einrichtung einstellbaren maximalen Pumpenfluss zeigt, das der maximale Pumpenfluss geringer wird, wenn der Parameter % ECLS_O2 um den ersten vorbestimmten Betrag verringert wird. Der maximale Pumpenfluss entspricht aber maximalen Fluss an Blut, das im extrakorporalen ECLS Kreislauf fließen kann und dementsprechend mit Sauerstoff angereichert wird. Das bedeutet, dass die Rahmenbedingungen für die extrakorporale Unterstützung durch die ECLS-Einrichtung 50 enger gesteckt werden und dafür die Rahmenbedingungen für die Unterstützung durch Beatmungseinrichtung 20 lockerer gesteckt werden.

Nach Schritt 208 gelangt man zu dem mit 210 bezeichneten Punkt 2 in Fig. 4. An diesem Punkt geht der Ablauf weiter zum Ventilationsmodul 300 in Fig. 1. Fig. 5 zeigt ein Flussdiagramm, das weitere Details der in dem Ventilations-Modul 300 ablaufenden Prozeduren verdeutlicht.

Zunächst wird wieder eine vorbestimmte Zeit abgewartet (Schritt 302), in dem gezeigten Beispiel 30s. Nach Ablauf dieser Zeit wird in Schritt 304 überprüft, ob die Ventilation des Blutkreislaufs ausreichend ist. Dies kann anhand eines oder mehrerer der bereits geschilderten Sensoren erfolgen, etwa durch eine laufend erfolgende Analyse der Blutgaswerte mit Bestimmung der Kohlendioxidkonzentration im Blut PaCO2 bzw. der entsprechenden Kohlendioxidsättigung SaCO2.

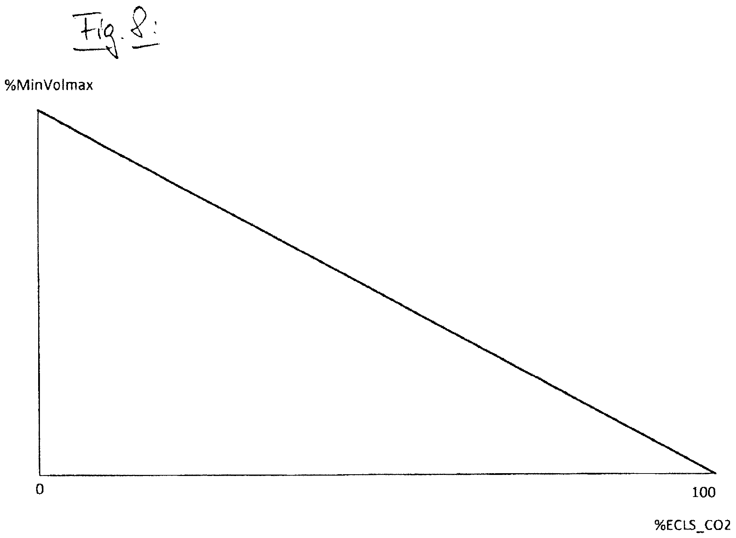

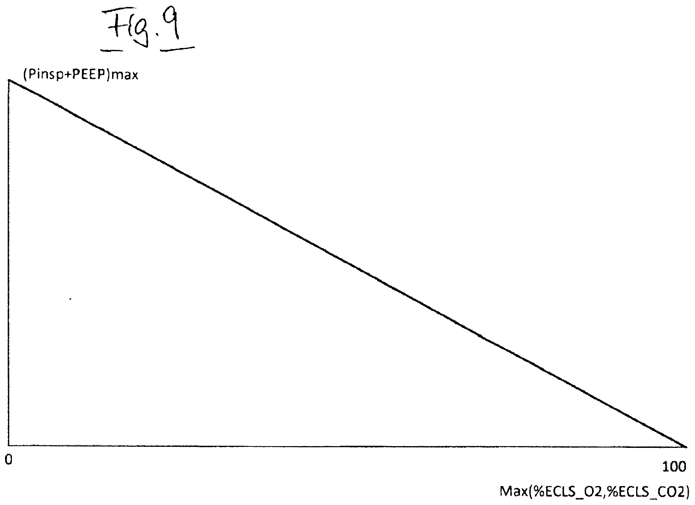

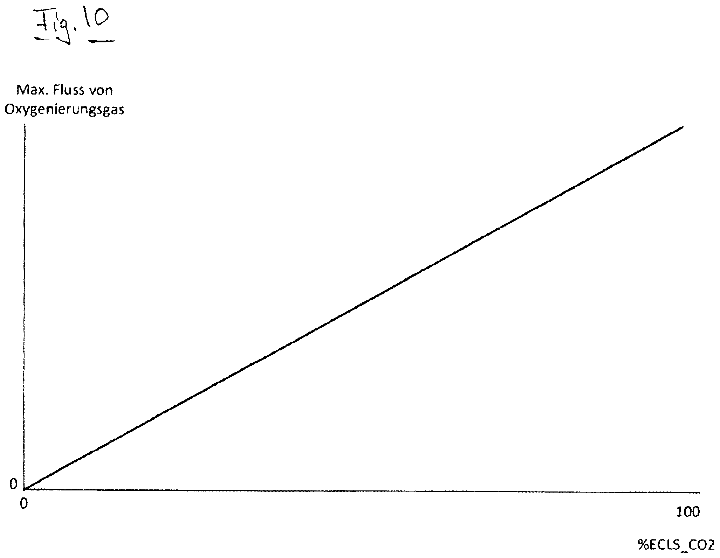

Auch eine Messung des CO2-Gehalts in der ausgeatmeten Luft, in Fig. 1 und 2 als PetCO2, kann herangezogen werden. Falls gewünscht kann eine solche Messung durch nur sporadisch mittels Blutgasanalyse ermittelte Werte für Pa-CO2 oder SaCO2 ergänzt werden. Ergibt die Bestimmung in Schritt 302, dass die Kohlendioxidkonzentration bzw. Kohlendioxidsättigung in einem erwünschten Bereich liegt, dann wird in Schritt 304 der Parameter %ECLS_CO2, der den Grad der extrakorporalen Unterstützung bei der Ventilation ausdrückt, um einen vorbestimmten zweiten Betrag verringert, in dem gezeigten Beispiel um 0,05%. Ab diesem Zeitpunkt verändern sich relativen Anteile der Unterstützung beim Blutgasaustausch zwischen extrakorporaler Blutgasaustauschunterstützung mittels ECLS-Einrichtung 50 und Unterstützung mittels Überdruckbeatmung. Dies lässt sich anhand des Schaubilder in Fig. 8 bis 10 ablesen: Dem in Fig. 8 gezeigten Diagramm, das qualitativ den Zusammenhang zwischen dem Grad der extrakorporalen Unterstützung bei der Ventilation %ECLS_CO2 und dem maximalen Minutenvolumen bei der Überdruckbeatmung %MinVolMax zeigt, ist zu entnehmen, dass die Verringerung des Parameters %ECLS_CO2um den zweiten vorbestimmten Betrag einerseits das für die Beatmungseinrichtung 20 vorgegebene maximale Minutenvolumen %MinVolMax erhöht. Andererseits entnimmt man dem Diagramm gem. Fig. 9, das qualitativ den Zusammenhang zwischen dem Maximum aus Grad der extrakorporalen Unterstützung bei der Oxygenierung % ECLS_O2 und Grad der extrakorporalen Unterstützung bei der Ventilation %ECLS_CO2einerseits sowie dem maximalen Atemwegsdruck (Pinsp+PEEP)max bei der Überdruckbeatmung zeigt, dass auch der maximale Atemwegsdruck (Pinsp+PEEP)max bei der Überdruckbeatmung größer wird, wenn sich beide Parameter, %ECLS O2 um den ersten vorbestimmten Betrag und %ECLS_CO2um den zweiten vorbestimmten Betrag, verringert haben. Sowohl eine Erhöhung des maximalen Minutenvolumens als auch eine Erhöhung des maximal erlaubten Atemwegsdrucks erlauben einen größeren Einfluss der Überdruckbeatmung an der insgesamt durch das Beatmungssystem ausgeübten Unterstützung beim Blutgasaustausch. Darüber hinaus zeigt die Fig. 10, das die Verringerung des Parameters %ECLS_CO2um den zweiten vorbestimmten Betrag noch einen weiteren Effekt hat. Dem in Fig. 10 gezeigten ein Diagramm lässt sich entnehmen, dass qualitativ der in der ECLS-Einrichtung einstellbare maximalen Fluss von Oxygenierungsgas geringer wird, je geringer der Grad der extrakorporalen Unterstützung bei der Ventilation %ECLS_CO2 wird. Damit wird bei Verringerung des Parameters %ECLS_CO2 aber auch der insgesamt mittels ECLS erzielbare Blutgasaustausch geringer, weil die Rahmenbedingungen für die extrakorporale Unterstützung durch die ECLS-Einrichtung enger gesteckt werden. Dafür werden die Rahmenbedingungen für die Unterstützung durch Überdruckbeatmung lockerer gesteckt.

Insgesamt führt dies dazu, dass im Punkt 310 insgesamt der Einfluss der Überdruckbeatmung auf Kosten des Einflusses des extrakorporalen Blutgasaustauschs gestiegen ist. Dies, obwohl an sich die gesteckten Zielwerte hinsichtlich Anreicherung des Blutes mit Sauerstoff bzw. Abreicherung des Blutes von CO2 bei der zu Anfang der Prozedur gewählten Einstellung erfüllt werden konnten.

Am mit dem Bezugszeichen 310 bezeichneten Punkt 1 in Fig. 5 angelangt, geht der Ablauf wieder zurück zu dem ebenfalls mit 310 bezeichnet Punkt 1 in Fig. 4. D.h. es folgt eine erneute Wartezeit mit sich anschließender neuer Erfassung des Zustands hinsichtlich Oxygenierung und ggf. Neueinstellung des Parameters %ECLS_O2.

Insgesamt bedeutet der beschriebene Ablauf, dass das Beatmungssystem 10 eine Tendenz hat, sich aus einem gewählten Anfangszustand heraus in einer Richtung zu entwickeln, in der die Überdruckbeatmung immer mehr Einfluss gewinnt und der extrakorporale Blutgasaustausch immer mehr Bedeutung verliert, dies zumindest solange, wie durch den kombinierten Effekt der Überdruckbeatmung und des extrakorporalen Blutgasaustauschs vorgegebene Zielwerte hinsichtlich Oxygenierung und Ventilation erreicht werden können. Das Beatmungssystem 10 stellt sich also von sich aus, ohne dass Eingriffe von außen erforderlich wären, in einen Zustand ein, bei dem die Überdruckbeatmung einen möglichst großen Einfluss nimmt und der extrakorporale Blutgasaustausch gerade soweit unterstützt, wie das notwendig ist. Diese Entwicklung wird dabei von der ECLS-Einrichtung 50 angetrieben, nicht jedoch von der Beatmungseinrichtung 20. Das führt im Endergebnis zu einer allmählichen, dafür stetigen Entwicklung mit wenig bis gar keinen Rückkopplungen auf die ECLS-Einrichtung 50 durch die sich jeweils an von der ECLS-Einrichtung 50 vorgegebene Rahmenbedingungen anpassende Beatmungseinrichtung 20.

Sollte der Fall eintreten, dass die in Schritt 206 erfasste Konzentration von Sauerstoff im Blut nicht dem gewünschten Zielwert entspricht, sondern vielmehr zu niedrig oder zu hoch liegt, dann ist vorgesehen, dass der den Grad der extrakorporalen Unterstützung der Oxygenierung anzeigende Parameter % ECLS_O2 um einen dritten Betrag verringert wird, der größer ist als der erste Betrag (Schritt 212), oder um einen vierten Betrag erhöht wird (Schritt 214). Der dritte Betrag ist größer als der erste Betrag, so dass der Anteil der Überdruckbeatmung in der Folge noch schneller ansteigt, als bei bloßem Erreichen des gewünschten Zielwerts für die Sauerstoffkonzentration im Blut. Umgekehrt führt die Erhöhung von %ECLS_O2 um den vierten Betrag dazu, dass die Unterstützung durch Überdruckbeatmung in der Folge nicht mehr größer wird, sondern umgekehrt kleiner wird. Das trägt der Tatsache Rechnung, dass in einer solchen Situation der Zustand des Patienten eine weitere Erhöhung des Anteils der Überdruckbeatmung nicht zulässt. Sollte die angestrebte Sauerstoffkonzentration drastisch verfehlt werden, wird auf einen Notfall geschlossen mit der Folge dass der Parameter %ECLS O2 drastisch erhöht wird, im dem Beispiel um 10 % (Schritt 216), damit in dem extrakorporalen Kreislauf ein ausreichender Blutgasaustausch sicherstellt werden kann.

Ähnliche Mechanismen greifen auch für das Ventilationsmodul 300. Sollte der Fall eintreten, dass die in Schritt 302 erfasste Konzentration von Kohlendioxid im Blut nicht dem gewünschten Zielwert entspricht, sondern vielmehr zu hoch oder zu niedrig liegt, dann ist vorgesehen, dass der den Grad der extrakorporalen Unterstützung der Ventilation anzeigende Parameter %ECLS_CO2um einen fünften Betrag verringert wird, der größer ist als der zweite Betrag (Schritt 306), oder um einen sechsten Betrag erhöht wird (Schritt 308). Der fünfte Betrag ist größer als der zweite Betrag, so dass der Anteil der Überdruckbeatmung in der Folge noch schneller ansteigt, als bei bloßem Erreichen des gewünschten Zielwerts für die Kohlendioxidkonzentration im Blut. Umgekehrt führt die Erhöhung von %ECLS_CO2 um den sechsten Betrag dazu, dass die Unterstützung durch Überdruckbeatmung in der Folge nicht mehr größer wird, sondern umgekehrt kleiner wird. Das trägt der Tatsache Rechnung, dass in einer solchen Situation der Zustand des Patienten eine weitere Erhöhung des Anteils der Überdruckbeatmung nicht zulässt.

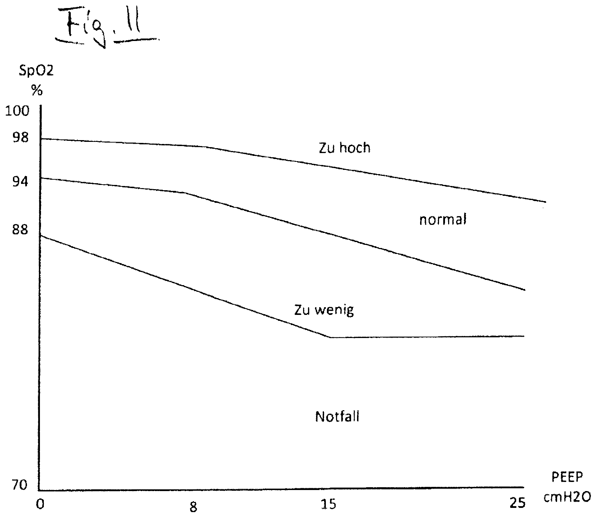

Fig. 11 zeigt ein Diagramm, das in qualitativer Weise andeutet, welche Werte für den mittels Pulsoxymetrie gemessenen Sauerstoffsättigungswert SpO2 bei jeweils herrschendem positiven endexpiratorischem Druck PEEP als zu hoch, zu niedrig, normal oder gar als Notfall angesehen werden und in der Abfrage gem. Fig. 4 zu entsprechenden Veränderungen des Parameters %ECLS_O2führen.

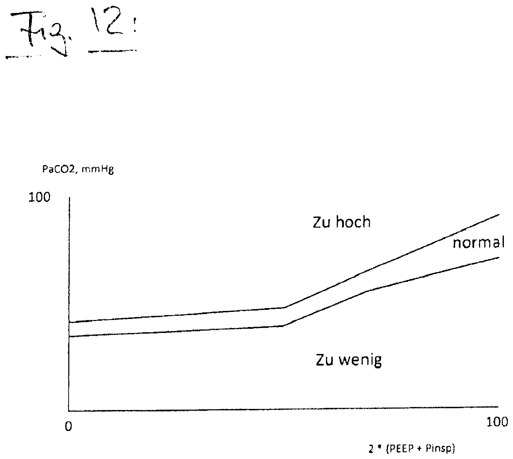

Fig. 12 zeigt ein Diagramm, das in qualitativer Weise andeutet, welche Werte für die im arteriellen Blut bestimmte Konzentration von CO2 PaCO2 bei jeweils herrschendem maximalem Atemwegsdruck (Pinsp + PEEP) als zu hoch, zu niedrig oder normal und in der Abfrage gem. Fig. 5 zu entsprechenden Veränderungen des Parameterss %ECLS_CO2 führen.

Bei allen gezeigten Diagrammen, insbesondere den in den Fig. 6 bis 12 dargestellten Zusammenhängen ist zu beachten, dass jeweils nur ein qualitativer Verlauf angedeutet werden soll. Quantitative Aussagen sollen damit nicht nicht gemacht werden. Gleichermaßen kann stellt der dargestellte lineare Verlauf der Beziehungen nur eine Vereinfachung dar. Der wahre Verlauf kann in Teilabschnitten oder sogar ganz von dem linearen Verlauf abweichen. Wichtig ist die jeweils angesprochene ansteigende oder abfallende Tendenz der jeweiligen Größen mit ansteigendem Wert von % ECLS_O2 bzw. %ECLS_CO2.

## Patentansprüche

1. Beatmungsystem zur Unterstützung des Blutgasaustauschs mittels maschineller Beatmung und extrakorporalem Blutgasaustausch (10), umfassend
eine Beatmungseinrichtung (20) zur maschinellen Beatmung der Lunge ei nes Patienten (12), und
eine ECLS (extracorporal lung support) Einrichtung (50) zum extrakorporalen Blutgasaustausch, wobei das System (10) dazu ausgebildet ist, eine maschinelle Atmungsunterstützung durch die Beatmungseinrichtung (20) einerseits und einen extrakorporalen Blutgasaustausch durch die ECLS-Einrichtung (50) andererseits in koordinierter Weise automatisiert durchzuführen, um den Gasaustausch im Blutkreislauf des Patienten zu unterstützen,
wobei die ECLS-Einrichtung (50) ein Niveau des extrakorporalen Blutgasaustauschs vorgibt und die Beatmungseinrichtung (20) sich anhand des von der ECLS-Einrichtung (50) vorgegebenen Niveaus des extrakorporalen Blutgasaustauschs automatisiert auf ein Niveau der maschinellen Atmungsunterstützung einstellt;
**dadurch gekennzeichnet, dass** dem durch die ECLS-Einrichtung (50) vorgegebenen Niveau des extrakorporalen Blutgasaustauschs ein Grad der extrakorporalen Unterstützung bei der Oxygenierung (%ECLS_O2), d.h. bei der Anreicherung des Blutes mit Sauerstoff, zugeordnet ist, welcher einen maximalen positiven endexpiratorischen Druck (PEEPmax) für die maschinelle Beatmung festlegt.

2. System (10) nach Anspruch 1, wobei das Niveau des extrakorporalen Blutgasaustauschs durch die ECLS-Einrichtung (50) automatisiert oder manuell vorwählbar ist.

3. System (10) nach Anspruch 2, wobei die ECLS-Einrichtung (50) einen Zielwert für das Niveau des extrakorporalen Blutgasaustauschs vorgibt; wobei insbesondere die Beatmungseinrichtung (20)für eine jeweils erfolgte Einstellung des Niveaus des extrakorporalen Blutgasaustauschs durch die ECLS-Einrichtung (50) die Atmungsunterstützung durch Überdruckbeatmung automatisch steuert/regelt wobei insbesondere die Beatmungseinrichtung (20) dazu ausgebildet ist, in automatisierter Weise und im Rahmen vorgegebener Beatmungsparameter einen durch die Beatmungseinrichtung (20) einzustellenden Beatmungszustand auszuwählen und die Beatmungseinrichtung (20) derart zu steuern/regeln, dass sie den ausgewählten Beatmungszustand einnimmt; oder
wobei die vorgegebenen Beatmungsparameter sich aus dem durch die ECLS-Einrichtung (50) vorgegebenen Niveau des extrakorporalen Blutgasaustauschs ableiten.

4. System (10) nach einem der Ansprüche 1 bis 3, wobei insbesondere der maximale positive endexpiratorische Druck (PEEPmax) mit abnehmendem Grad der extrakorporalen Unterstützung bei der Oxygenierung (%ECLS_O2) ansteigt.

5. System (10) nach Anspruch 4, wobei der Grad der extrakorporalen Unterstützung bei der Oxygenierung (%ECLS_O2) einen Maximalwert für den Fluss des durch die ECLS-Einrichtung dem Patienten entnommenen Blutes (Max. Pumpenfluss) festlegt, wobei insbesondere der Maximalwert für den Fluss des durch die ECLS-Einrichtung dem Patienten entnommenen Blutes (max. Pumpenfluss) mit zunehmendem Grad der extrakorporalen Unterstützung bei der Oxygenierung (%ECLS_O2) ansteigt.

6. System (10) nach einem der Ansprüche 1 bis 5, wobei dem durch die ECLS-Einrichtung (50) vorgegebenen Niveau des extrakorporalen Blutgasaustauschs ein Grad der extrakorporalen Unterstützung bei der Ventilation (%ECLS_CO2), d.h. bei der Entfernung von CO2 aus dem Blut, zugeordnet ist, wobei insbesondere der Grad der extrakorporalen Unterstützung bei der Ventilation (%ECLS_CO2) ein maximales Minutenvolumen (%MinVolMax) für die maschinelle Beatmung festlegt, wobei insbesondere das maximale Minutenvolumen (%MinVolMax) mit abnehmendem Grad der extrakorporalen Unterstützung bei der Ventilation (%ECLS_CO2) ansteigt.

7. System (10) nach Anspruch 6, wobei der Grad der extrakorporalen Unterstützung bei der Ventilation (%ECLS_CO2) einen maximalen Atemwegsdruck ((Pinsp+PEEP)max) für die maschinelle Beatmung festlegt, wobei insbesondere der maximale Atemwegsdruck ((Pinsp+PEEP)max) mit abnehmendem Grad des Maximalwerts aus extrakorporaler Unterstützung bei der Oxygenierung (%ECLS_O2) und extrakorporaler Unterstützung bei der Ventilation (%ECLS_CO2) ansteigt.

8. System (10) nach 6 oder 7, wobei der Grad der extrakorporalen Unterstützung bei der Ventilation (%ECLS_CO2) einen Maximalwert des Flusses von Oxygenierungsgas, das dem dem Blutkreislauf des Patienten entnommenen Blut durch die ECLS Einrichtung zugeführt wird, festlegt, wobei insbesondere der Maximalwert des Flusses von Oxygenierungsgas, das dem dem Blutkreislauf des Patienten entnommenen Blut durch die ECLS Einrichtung zugeführt wird, mit zunehmendem Grad der extrakorporalen Unterstützung bei der Ventilation (%ECLS_O2) ansteigt.

9. System (10) nach einem der Ansprüche 1 bis 8, wobei die ECLS-Einrichtung (50) - bei einem jeweiligen Niveau des extrakorporalen Blutgasaustauschs - nach Ablauf einer vorbestimmten Zeit überprüft, ob bei dem Niveau des extrakorporalen Blutgasaustauschs durch die Beatmungseinrichtung (20) und die ECLS-Einrichtung (50) gemeinsam ein vorbestimmter Zielzustand für den Blutgasaustausch erreicht wird, wobei insbesondere der vorbestimmte Zielzustand für den Blutgasaustausch durch eine Größe gekennzeichnet ist, die die Konzentration von O2 im Blutkreislauf kennzeichnet und/oder durch eine Größe gekennzeichnet ist, die die Konzentration von CO2 im Blutkreislauf kennzeichnet.

10. System (10) nach Anspruch 9, wobei die ECLS-Einrichtung (50) bei Erreichen des vorbestimmten Zielzustands das Niveau des extrakorporalen Blutgasaustauschs verringert.

11. System (10) nach 9 oder 10, wobei die ECLS-Einrichtung (50) bei Erreichen des vorbestimmten Zielzustands, insbesondere bei Erreichen des vorgegebenen Werts der Konzentration von O2 im Blutkreislauf, den Grad der extrakorporalen Unterstützung bei der Oxygenierung (%ECMO_O2), um einen ersten vorbestimmten Betrag verringert.

12. System (10) nach einem der Ansprüche 9 bis 11, wobei die ECLS-Einrichtung (50) bei Erreichen des vorbestimmten Zielzustands, insbesondere bei Erreichen des vorgegebenen Werts der Konzentration von CO2 im Blutkreislauf, den Grad der extrakorporalen Unterstützung bei der Ventilation (%ECMO_CO2), um einen zweiten vorbestimmten Betrag verringert.

13. System (10) nach einem der Ansprüche 9 bis 12, wobei die ECLS-Einrichtung (50) in wiederkehrenden Zeitabständen überprüft, ob bei dem jeweiligen Niveau des extrakorporalen Blutgasaustauschs durch die Beatmungseinrichtung (20) und die ECLS-Einrichtung (50) gemeinsam ein vorbestimmter Zielwert für den Blutgasaustausch erreicht wird, wobei insbesondere der Zeitabstand für die Überprüfung durch die ECLS-Einrichtung (50) größer ist, insbesondere deutlich größer ist, als die Zeitkonstante der Beatmungseinrichtung (20).

14. System (10) nach einem der Ansprüche 1 bis 13 wobei die ECLS-Einrichtung (50) von einem voreingestellten Startwert für das Niveau des extrakorporalen Blutgasaustauschs ausgeht, wobei insbesondere der Startwert einem maximalen Niveau für das Niveau des extrakorporalen Blutgasaustauschs entspricht.

15. System (10) nach Anspruch 14, welches den Startwert als Bezugsgröße für die Verringerung bzw. Erhöhung des des Niveaus der extrakorporalen Blutgasaustauschs nimmt.

## Claims

1. A ventilation system for supporting the blood gas exchange by means of mechanical ventilation and extracorporeal blood gas exchange (10), comprising:
a ventilation device (20) for mechanical ventilation of the lungs of a patient (12), and
an ECLS (extracorporeal lung support) device (50) for extracorporeal blood gas exchange,
wherein the system (10) is designed to perform mechanical respiratory support by the ventilation device (20) on the one hand and extracorporeal blood gas exchange by the ECLS device (50) on the other hand, in coordinated automated manner in order to support the gas exchange in the blood circulation of the patient,
wherein the ECLS device (50) sets a level of the extracorporeal blood gas exchange and the ventilation device (20), on the basis of the level of the extracorporeal blood gas exchange set by the ECLS device (50), adjusts in automated manner to a level of mechanical respiratory support;
**characterised in that** the level of extracorporeal blood gas exchange set by the ECLS device (50) is associated with a degree of extracorporeal support in oxygenation (%ECLS_O2), i.e. in the enrichment of the blood with oxygen, which determines a maximum positive end-expiratory pressure (PEEPmax) for mechanical ventilation.

2. The system (10) of claim 1,
wherein the level of the extracorporeal blood gas exchange can be preselected by the ECLS device (50) in automated or manual manner.

3. The system (10) of claim 2,
wherein the ECLS device (50) sets a target value for the level of the extracorporeal blood gas exchange; wherein in particular the ventilation device (20), for a respective effected setting of the level of the extracorporeal blood gas exchange by the ECLS device (50), automatically controls the respiratory support by positive-pressure ventilation, wherein in particular the ventilation device (20) is designed to select, in automated manner and in the scope of set ventilation parameters, a ventilation state to be set by the ventilation device (20), and to control the ventilation device (20) such that the latter assumes the selected ventilation state; or
wherein the set ventilation parameters are derived from the level of the extracorporeal blood gas exchange set by the ECLS device (50).

4. The system (10) of any of claims 1 to 3,
wherein in particular the maximum positive end-expiratory pressure (PEEPmax) increases with a decreasing degree of extracorporeal support in oxygenation (%ECLS_O2).

5. The system (10) of claim 4,
wherein the degree of extracorporeal support in oxygenation (%ECLS_O2) determines a maximum value for the flow of the blood taken from the patient by the ECLS device (max. pump flow), wherein in particular the maximum value for the flow of the blood taken from the patient by the ECLS device (max. pump flow) increases with an increasing degree of extracorporeal support in oxygenation (%ECLS_O2).

6. The system (10) of any of claims 1 to 5,
wherein the level of the extracorporeal blood gas exchange set by the the ECLS device (50) is associated with a degree of extracorporeal support in ventilation (%ECLS_CO2), i.e. in the removal of CO2 from the blood, wherein in particular the degree of extracorporeal support in ventilation (%ECLS_CO2) determines a maximum minute volume (%MinVolMax) for mechanical ventilation, wherein in particular the maximum minute volume (%MinVolMax) increases with a decreasing degree of extracorporeal support in ventilation (%EOLS_CO2).

7. The system (10) of claim 6,
wherein the degree of extracorporeal support in ventilation (%ECLS_CO2) determines a maximum airway pressure ((Pinsp+PEEP)max) for mechanical ventilation, wherein in particular the maximum airway pressure ((Pinsp+PEEP)max) increases with a decreasing degree of the maximum value of extracorporeal support in oxygenation (%ECLS_O2) and extracorporeal support in ventilation (%EOLS_CO2).

8. The system (10) of claim 6 or 7,
wherein the degree of extracorporeal support in ventilation (%ECLS_CO2) determines a maximum value of the flow of oxygenation gas that the ECLS device supplies to the blood taken from the patient's blood circulation, wherein in particular the maximum value of the flow of oxygenation gas that the ECLS device supplies to the blood taken from the patient's blood circulation, increases with an increasing degree of extracorporeal support in ventilation (%ECLS_O2).

9. The system (10) of any of claims 1 to 8,
wherein the ECLS device (50) - at a respective level of the extracorporeal blood gas exchange - examines after expiration of a predetermined period of time whether, at the level of the extracorporeal blood gas exchange, a predetermined target state for the blood gas exchange is reached by the ventilation device (20) and the ECLS device (50) together, wherein in particular the predetermined target state for the blood gas exchange is **characterized by** a parameter that defines the concentration of O2 in the blood circulation and/or is **characterized by** a parameter that defines the concentration of CO2 in the blood circulation.

10. The system (10) of claim 9,
wherein the ECLS device (50), upon reaching the predetermined target state, reduces the level of the extracorporeal blood gas exchange.

11. The system (10) of claim 9 or 10,
wherein the ECLS device (50), upon reaching the predetermined target state, in particular upon reaching the set value of the concentration of O2 in the blood circulation, reduces the degree of extracorporeal support in oxygenation (%ECMO_O2) by a first predetermined amount.

12. The system (10) of any of claims 9 to 11,
wherein the ECLS device (50), upon reaching the predetermined target state, in particular upon reaching the set value of the concentration of CO2 in the blood circulation, reduces the degree of extracorporeal support in ventilation (%ECLS_CO2) by a second predetermined amount.

13. The system (10) of any of claims 9 to 12,
wherein the ECLS device (50) examines in recurrent intervals of time whether, at the respective level of the extracorporeal blood gas exchange, a predetermined target value for the blood gas exchange is reached by the ventilation device (20) and the ECLS device (50) together, wherein in particular the time interval for the examination by the ECLS device (50) is greater, in particular clearly greater, than the time constant of the ventilation device (20).

14. The system (10) of any of claims 1 to 13,
wherein the ECLS device (50) starts from a preset starting value for the level of the extracorporeal blood gas exchange, wherein in particular the starting value corresponds to a maximum level for the level of the extracorporeal blood gas exchange.

15. The system (10) of claim 14,
which makes use of the starting value as reference value for the reduction and increase, respectively, of the level of the extracorporeal blood gas exchange.

## Revendications

1. Système d'assistance respiratoire destiné à supporter l'échange entre les gaz du sang au moyen d'une assistance respiratoire mécanique et d'un échange extracorporel (10) entre les gaz du sang, comprenant :
un mécanisme d'assistance respiratoire (20) destiné à l'assistance respiratoire mécanique des poumons d'un patient (12) ; et
un dispositif ECLS (extracorporeal lung support/assistance pulmonaire extracorporelle) (50) destiné à l'échange extracorporel entre les gaz du sang ;
dans lequel le système (10) est réalisé dans le but d'apporter une assistance respiratoire mécanique par l'intermédiaire du mécanisme d'assistance respiratoire (20) d'une part et de mettre en œuvre un échange extracorporel entre les gaz du sang par l'intermédiaire du mécanisme ECLS (50) d'autre part, de façon automatisée et de manière coordonnée, à des fins de support pour l'échange gazeux dans la circulation sanguine du patient ;
dans lequel le mécanisme ECLS (50) prédéfinit un niveau de l'échange extracorporel entre les gaz du sang et le mécanisme d'assistance respiratoire (20) se règle de manière automatisée, sur base du niveau de l'échange extracorporel entre les gaz du sang, prédéfini par le mécanisme ECLS (50), à un niveau de l'assistance respiratoire mécanique ;
**caractérisé en ce qu'**un degré de l'assistance extracorporelle lors de l'oxygénation (%ECLS_O2), c'est-à-dire lors de l'enrichissement du sang avec de l'oxygène, est attribué au niveau prédéfini de l'échange extracorporel entre les gaz du sang, prédéfini par le mécanisme ECLS (50), degré qui détermine une pression positive maximale en fin d'expiration (PEEPmax) pour l'assistance respiratoire mécanique.

2. Système (10) selon la revendication 1, dans lequel le niveau de l'échange extracorporel entre les gaz du sang peut faire l'objet d'une sélection préalable de façon manuelle ou de manière automatisée par l'intermédiaire du mécanisme ECLS (50).

3. Système (10) selon la revendication 2, dans lequel le mécanisme ECLS (50) prédéfinit une valeur cible pour le niveau de l'échange extracorporel entre les gaz du sang ; dans lequel, en particulier, le mécanisme d'assistance respiratoire (20) commande/règle de manière automatique, pour un réglage du niveau de l'échange extracorporel entre les gaz du sang chaque fois mis en place, par l'intermédiaire du mécanisme ECLS (50), l'assistance respiratoire par l'intermédiaire d'une ventilation en pression positive ; dans lequel, en particulier, le mécanisme d'assistance respiratoire (20) est réalisé dans le but de sélectionner, de manière automatique et dans le cadre de paramètres d'assistance respiratoire prédéfinis, un état d'assistance respiratoire qui doit être réglé par l'intermédiaire du mécanisme d'assistance respiratoire (20), et dans le but de commander/régler le mécanisme d'assistance respiratoire (20) d'une manière telle que ce dernier adopte l'état d'assistance respiratoire qui a été sélectionné ; ou dans lequel les paramètres d'assistance respiratoire prédéfinis dérivent du niveau de l'échange extracorporel entre les gaz du sang, prédéfini par le mécanisme ECLS (50).

4. Système (10) selon l'une quelconque des revendications 1 à 3, dans lequel, en particulier, la pression positive maximale en fin d'expiration (PEEPmax) augmente de manière proportionnelle à la diminution du degré de l'assistance extracorporelle lors de l'oxygénation (%ECLS_O2).

5. Système (10) selon la revendication 4, dans lequel le degré de l'assistance extracorporelle lors de l'oxygénation (%ECLS_02) détermine une valeur maximale pour le flux du sang prélevé du patient (Max. Pumpenfluss) par l'intermédiaire du mécanisme ECLS ; dans lequel, en particulier, la valeur maximale pour le flux du sang prélevé du patient (Max. Pumpenfluss) par l'intermédiaire du mécanisme ECLS augmente de manière proportionnelle à l'augmentation du degré de l'assistance extracorporelle lors de l'oxygénation (%ECLS_O2).

6. Système (10) selon l'une quelconque des revendications 1 à 5, dans lequel un degré de l'assistance extracorporelle lors de la ventilation (%ECLS_CO2), c'est-à-dire lors de l'élimination de CO2 à partir du sang, est attribué au niveau de l'échange extracorporel entre les gaz du sang, prédéfini par l'intermédiaire du mécanisme ECLS (50) ; dans lequel, en particulier, le degré de l'assistance extracorporelle détermine, lors de la ventilation (%ECLS_C02), un volume maximal par minute (%MinVolMax) pour l'assistance respiratoire mécanique ; dans lequel, en particulier, le volume maximal par minute (%MinVolMax) augmente de manière proportionnelle avec la diminution du degré de l'assistance extracorporelle lors de la ventilation (%ECLS_C02).

7. Système (10) selon la revendication 6, dans lequel le degré de l'assistance extracorporelle lors de la ventilation (%ECLS_CO2) détermine une pression maximale des voies respiratoires ((Pinsp+PEEP)max) pour l'assistance respiratoire mécanique ; dans lequel, en particulier, la pression maximale des voies respiratoires ((Pinsp+PEEP)max) augmente de manière proportionnelle à la diminution du degré de la valeur maximale que l'on obtient à partir de l'assistance extracorporelle lors de l'oxygénation (%ECLS_O2) et à partir de l'assistance extracorporelle lors de la ventilation (%EOLS_CO2).

8. Système (10) selon la revendication 6 ou 7, dans lequel le degré de l'assistance extracorporelle lors de la ventilation (%ECLS_CO2) détermine une valeur maximale du flux du gaz d'oxygénation qui est ajouté par l'intermédiaire du mécanisme ECLS au sang prélevé à la circulation sanguine du patient ; dans lequel, en particulier, la valeur maximale du flux du gaz d'oxygénation qui est ajouté par l'intermédiaire du mécanisme ECLS au sang prélevé à la circulation sanguine du patient augmente de manière proportionnelle à l'augmentation du degré de l'assistance extracorporelle lors de la ventilation (%ECLS_CO2).

9. Système (10) selon l'une quelconque des revendications 1 à 8, dans lequel le mécanisme ECLS (50) - à un niveau respectif de l'échange extracorporel entre les gaz du sang - après un laps de temps prédéfini, vérifie le fait de savoir si, au niveau de l'échange extracorporel entre les gaz du sang, par l'intermédiaire du mécanisme d'assistance respiratoire (20) et du mécanisme ECLS (50) de manière conjointe, on atteint un état cible prédéfini pour l'échange entre les gaz du sang ; dans lequel, en particulier, l'état cible prédéfini pour l'échange entre les gaz du sang est **caractérisé par** une valeur qui caractérise la concentration de 02 dans la circulation sanguine et/ou est **caractérisé par** une valeur qui caractérise la concentration de CO2 dans la circulation sanguine.

10. Système (10) selon la revendication 9, dans lequel le mécanisme ECLS (50) réduit, lorsqu'on atteint l'état cible prédéfini, le niveau de l'échange extracorporel entre les gaz du sang.

11. Système (10) selon la revendication 9 ou 10, dans lequel le mécanisme ECLS (50), lorsqu'on atteint l'état cible prédéfini, en particulier lorsqu'on atteint la valeur prédéfinie de la concentration de 02 dans la circulation sanguine, réduit le degré de l'assistance extracorporelle lors de l'oxygénation (%ECMO_O2) d'une première valeur prédéfinie.

12. Système (10) selon l'une quelconque des revendications 9 à 11, dans lequel le mécanisme ECLS (50), lorsqu'on atteint l'état cible prédéfini, en particulier lorsqu'on atteint la valeur prédéfinie de la concentration de CO2 dans la circulation sanguine, réduit le degré de l'assistance extracorporelle lors de la ventilation (%ECMO_CO2) d'une deuxième valeur prédéfinie.

13. Système (10) selon l'une quelconque des revendications 9 à 12, dans lequel le mécanisme ECLS (50) vérifie, à des intervalles de temps récurrents, le fait de savoir si, au niveau respectif de l'échange extracorporel entre les gaz du sang, par l'intermédiaire du mécanisme d'assistance respiratoire (20) et du mécanisme ECLS (50) de manière conjointe, on atteint une valeur cible prédéfinie pour l'échange entre les gaz du sang ; dans lequel, en particulier, l'intervalle de temps pour la vérification par l'intermédiaire du mécanisme ECLS est supérieur, en particulier nettement supérieur à la constante de temps du mécanisme d'assistance respiratoire (20).

14. Système (10) selon l'une quelconque des revendications 1 à 13, dans lequel le mécanisme ECLS (50) se base sur une valeur de départ préréglée pour le niveau de l'échange extracorporel entre les gaz du sang ; dans lequel, en particulier, la valeur de départ correspond à un niveau maximal pour le niveau de l'échange extracorporel entre les gaz du sang.

15. Système (10) selon la revendication 14, qui prend la valeur de départ à titre de valeur de référence pour la réduction, respectivement l'augmentation du niveau de l'échange extracorporel entre les gaz du sang.
